(19) Europäisches Patentamt
European Patent Office
Office européen des brevets

(11) Veröffentlichungsnummer: **0 447 956 A1**

# (12) EUROPÄISCHE PATENTANMELDUNG

(21) Anmeldenummer: **91103900.6**

(22) Anmeldetag: **14.03.91**

(51) Int. Cl.5: **C12N 15/30**, C07K 15/00, C12N 1/21, C12P 21/00, A61K 39/015

(30) Priorität: **23.03.90 CH 970/90**

(43) Veröffentlichungstag der Anmeldung: **25.09.91 Patentblatt 91/39**

(84) Benannte Vertragsstaaten: **AT BE CH DE DK ES FR GB IT LI NL SE**

(71) Anmelder: **F. HOFFMANN-LA ROCHE AG**
**Postfach 3255**
**CH-4002 Basel(CH)**

(72) Erfinder: **Certa, Ulrich, Dr.**
**Steinbühlweg 21**
**CH-4123 Allschwil(CH)**
Erfinder: **Guttinger, Maria, Dr.**
**Unterer Rheinweg 130**
**CH-4057 Basel(CH)**
Erfinder: **Matile, Hugues, Dr.**
**Eisengasse 6**
**CH-4051 Basel(CH)**

(74) Vertreter: **Mezger, Wolfgang, Dr. et al**
**Grenzacherstrasse 124 Postfach 3255**
**CH-4002 Basel(CH)**

## (54) Plasmodium Sporozoitenantigen.

(57) Polypeptide, die in mindestens einem spezifischen Epitop mit einem Plasmodium falciparum Sporozoiten Antigen mit der in Fig. 1 gezeigten N-terminalen Aminosäuresequenz übereinstimmen, sowie ein Verfahren zu deren Herstellung sind offenbart. Ferner betrifft die Erfindung immunogene Kompositionen, die ein solches Polypeptid und ein geeignetes Adjuvans enthalten, eine DNA, die für ein solches Polypeptid kodiert, replizierbare mikrobielle Vektoren, die eine solche DNA enthalten, Mikroorganismen, die einen solchen replizierbaren Vektor enthalten sowie Antikörper gegen ein erfindungsgemässes Polypeptid. Verfahren zur Herstellung der immunogenen Kompositionen, der Mikroorganismen und der Antikörper sowie die Verwendung der Polypeptide und der immunogenen Kompositionen zur Immunisierung von Säugern gegen Malaria sind beansprucht.

FIG 1

```
                        5                   10                  15
                        |                   |                   |
  1 Met Asn Lys Val Asn Ala Val His Lys Ile Asn Ala Val Asp Lys
 16 Val Asn Ala Val Asn Lys Val Asn Ser Val Asn Lys Leu Asn Val
 31 Val Asn Lys Thr Asn Val Leu Ser Lys Leu Asn Ala Val Tyr Lys
 46 Val Asn Ser Val His Lys Met Asn Ala Val Asn Lys Val Asn Ala
 61 Val Asn Lys Val Asn Ala Val Asn Lys Val Asn Val Val Asn Lys
 76 Lys Asp Ile Leu Asn Lys Leu Asn Ala Leu Tyr Lys Met Asn Ala
 91 Val Tyr Lys Met Asn Ala Leu Asn Lys Val Ser Ala Val Asn Lys
106 Val Ser Ala Val Asn Lys Val Ser Ala Val Asn Lys Met Gly Ala
121 Val Asn Arg Val Asn Gly Val Asn Lys Val Asn Glu Val Asn Glu
136 Val Asn Glu Val Asn Glu Val Asn Met Val Asn Glu Val Asn Glu
151 Leu Asn Glu Val Asn Asn Val Asn Ala Val Asn Glu Val Asn Ser
166 Val Asn Glu Val Asn Glu Met Asn Glu Val Asn Lys Val Asn Glu
181 Leu Asn Glu Val Asn Glu Val Asn Asn Val Asn Glu Val Asn Asn
196 Val Asn Val Met Asn Asn Val Asn Glu Met Asn Asn Met Asn Glu
211 Met Asn Asn Val Asn Val Val Asn Glu Val Asn Asn Val Asn Glu
226 Val Asn Asn Val Asn Glu Met Asn Asn Val Asn Glu Met Asn Asn
241 Met Asn Glu Met Asn Asn Val Asn Val Val Asn Glu Val Asn Asn
256 Val Asn Glu Met Asn Asn Thr Asn Glu Leu Asn Glu Val Asn Glu
271 Val Asn Asn Val Asn Glu Val Asn Asp Val Asn Val Val Asn Glu
286 Val Asn Asn Val Asn Glu Met Asn Asn Met Asn Glu Leu Asn Glu
301 Val Asn Gly Val Asn Glu Val Asn Asn Thr Asn Glu Ile His Glu
316 Met Asn Asn Ile Asn Glu Val Asn Asn Thr Asn Glu Val Asn Asn
331 Thr Asn Glu Ile Tyr Glu Met Asn Asn Met Asn Asp Val Asn Asn
346 Thr Asn Glu Ile Asn Val Val Asn Ala Val Asn Glu Val Asn Lys
361 Val Asn Asp Ser Asn Asn Ser Asn Asp Ala Asn Glu Gly Asn Asn
376 Ala Asn Tyr Ser Asn Asp Ser Ser Asn Thr Asn Asn Asn Thr Ser
391 Ser Ser Thr Asn Asn Ser Asn Asn Asn Thr Ser Cys Ser Ser Gln
406 Asn Thr Thr Thr Ser Ser Glu Asn Asn Asp Ser Leu Glu Asn Lys
421 Arg Asn Glu Glu Asp Glu Asp Glu Glu Asp Asp Gln Lys Asp Thr
436 Gln Lys Glu Lys Asn Asn Leu Glu Gln Glu Asp Met Ser Pro Tyr
451 Glu Asp Arg Asn Lys Asn Asp Glu Lys Asn Ile Asn Glu Gln Asp
466 Lys Phe His Leu Ser Asn Asp Leu Gly Lys Ile Tyr Asp Thr Tyr
481 Asn Gln Gly Asp Glu Val Val Val Ser Lys Asn Lys Asp Lys Leu
496 Glu Lys His Leu Asn Asp Tyr Lys Ser Tyr Tyr Tyr Leu Ser Lys
511 Ala Thr Leu Met Asp Lys Ile Gly Glu Ser Gln Asn Asn Asn Asn
526 Tyr Asn Val Cys Asn Ser Asn Glu Leu Gly Thr Asn Glu Ser Ile
541 Lys Thr Asn Ser Asp Gln Asn Asp Asn Val Lys Glu Lys Asn Asp
556 Ser Asn Ile Phe Met Lys Met Ile Ile Ile Ile Arg Leu Met Ile
571 Met Ile Ile Met Ile Met Ile Ile Ile Trp Tyr Leu Lys Ile Leu
586 Gln Asp Lys Ile Ile Trp Arg Asn Lys Lys Val Glu Lys Thr Ser
601 Asn Ile Leu Asn Asn Phe Asp Asn Asn Gly Asn Asp Asn Asp Asn
616 Asp Asn Asp Asp Asn Asn Asp Asn Asp Asn Asn Asn Asn Asn Asn
631 Met Asn Asn Gln Tyr Asn Tyr Gln Glu Asn Asn Ile Asn Thr Asn
646 Tyr Asn Ile Leu Tyr Thr Pro Ser Asn Cys Gln Ile Gln Asn Asn
661 Ser Tyr Met Asn Thr Asn Glu Met Tyr Gln Pro Leu Tyr Asn Thr
676 Tyr Pro Ser Asn Arg Ile Gln Glu Asn Ser Thr Ile Asn Asn Asn
691 Ile Ile Asn Asp Ser Pro Tyr Met Asn Asn Asp Asn Thr Thr Asn
706 Asn Thr Phe Ile Ser Gly Met Asn
```

EP 0 447 956 A1

Malaria wird beim Menschen durch vier Spezies von Plasmodium verursacht, nämlich durch P. falciparum, P. vivax, P. ovale und P. malariae. Gemäss einem Bericht der Weltgesundheitsorganisation (WHO) aus dem Jahre 1986, gibt es weltweit fast 100 Millionen Fälle von Malariainfektionen. Davon haben etwa 1 Million, meistens Fälle von Kleinkindern, die mit P. falciparum infiziert sind, tödlichen Ausgang. Wegen dem Auftauchen von drogenresistenten Parasiten und von insektizidresistenten Moskitovektoren breitet sich die Malaria weiter aus. So berichteten die Gesundheitsbehörden Indiens 1962 von 100'000 Fällen von Malaria und 1980 schon von 3 Millionen Fällen, meistens verursacht durch P. vivax (vergl. Bruce-Chwatt, Essential Malariology, 2. Auflage, Heinemann, London [1986]). In den letzten Jahren haben neu entwickelte Methoden die Hoffnung genährt, dass es bald möglich sein wird Antimalaria-Vakzine herzustellen, die der wachsenden Ausbreitung der Malaria entgegen wirken können (Scaife, Genetic Engineering 7, 57-90 [1988]).

Der natürliche Lebenszyklus von P. falciparum umfasst drei verschiedene Stadien. Im ersten Stadium führen Moskitos während der Nahrungsaufnahme Sporozoiten in die Blutbahn von Wirbeltieren ein. Diese Sporozoiten wandern über die Blutbahn in die Leber und dringen in die Hepatozyten des Wirtes ein. Im zweiten Stadium entwickeln sich aus diesen Sporozoiten Merozoiten. Diese Merozoiten durchlaufen mehrere Multiplikationszyklen in Erythrozyten des Wirtes und entwickeln sich dann zu Gametozyten. Die Gametozyten, die das sexuelle Stadium des Parasiten darstellen, werden von Moskitos bei der Nahrungsaufnahme aufgenommen. Nach der Befruchtung im Darm des Insektes entwickeln sich aus den Gametozyten Sporozoiten, die dann in die Speicheldrüsen des Insektes wandern. Von dort aus kann der Zyklus von neuem beginnen.

Damit sind also Vakzine gegen die Sporozoitenform von Plasmodium falciparum die erste Verteidigungslinie gegen eine Infektion mit Malaria. In den letzten Jahren wurde das sog. CS-Protein aus Sporozoiten verschiedener Spezies von Plasmodium eingehend untersucht und seine Verwendung in Vakzinen geprüft (Nussenzweig et al., Adv. Immunol. 45, 283-334 [1989]). Obwohl Vakzinierungsversuche mit dem CS-Protein oder Derivaten davon einen teilweisen Schutz gegen eine spätere Malariainfektion erzeugten, kann noch nicht von einem Durchbruch bei der Entwicklung eines allgemein verwendbaren Malariavakzines gesprochen werden (Herrington et al., Nature 328, 257-259 [1987]). Es stellte sich daher die Aufgabe neue Sporozoitenantigene zu suchen, die in nativer oder abgeänderter Form zur Herstellung von neuen Antimalariavakzinen verwendet werden können.

Die vorliegende Anmeldung offenbart ein neues Sporozoitenantigen mit einem scheinbaren Molekulargewicht grösser 200 kDalton (kDa) mit der N-terminalen Aminosäuresequenz (I)

```
                          5                        10                        15
                          |                         |                         |
  1 Met Asn Lys Val Asn Ala Val His Lys Ile Asn Ala Val Asp Lys
 16 Val Asn Ala Val Asn Lys Val Asn Ser Val Asn Lys Leu Asn Val
 31 Val Asn Lys Thr Asn Val Leu Ser Lys Leu Asn Ala Val Tyr Lys
 46 Val Asn Ser Val His Lys Met Asn Ala Val Asn Lys Val Asn Ala
 61 Val Asn Lys Val Asn Ala Val Asn Lys Val Asn Val Val Asn Lys
 76 Lys Asp Ile Leu Asn Lys Leu Asn Ala Leu Tyr Lys Met Asn Ala
 91 Val Tyr Lys Met Asn Ala Leu Asn Lys Val Ser Ala Val Asn Lys
106 Val Ser Ala Val Asn Lys Val Ser Ala Val Asn Lys Met Gly Ala
121 Val Asn Arg Val Asn Gly Val Asn Lys Val Asn Glu Val Asn Glu
136 Val Asn Glu Val Asn Glu Val Asn Met Val Asn Glu Val Asn Glu
151 Leu Asn Glu Val Asn Asn Val Asn Ala Val Asn Glu Val Asn Ser
166 Val Asn Glu Val Asn Glu Met Asn Glu Val Asn Lys Val Asn Glu
181 Leu Asn Glu Val Asn Glu Val Asn Asn Val Asn Glu Val Asn Asn
196 Val Asn Val Met Asn Asn Val Asn Glu Met Asn Asn Met Asn Glu
211 Met Asn Asn Val Asn Val Val Asn Glu Val Asn Asn Val Asn Glu
226 Val Asn Asn Val Asn Glu Met Asn Asn Val Asn Glu Met Asn Asn
241 Met Asn Glu Met Asn Asn Val Asn Val Val Asn Glu Val Asn Asn
256 Val Asn Glu Met Asn Asn Thr Asn Glu Leu Asn Glu Val Asn Glu
271 Val Asn Asn Val Asn Glu Val Asn Asp Val Asn Val Val Asn Glu
286 Val Asn Asn Val Asn Glu Met Asn Asn Met Asn Glu Leu Asn Glu
301 Val Asn Gly Val Asn Glu Val Asn Asn Thr Asn Glu Ile His Glu
316 Met Asn Asn Ile Asn Glu Val Asn Asn Thr Asn Glu Val Asn Asn
331 Thr Asn Glu Ile Tyr Glu Met Asn Asn Met Asn Asp Val Asn Asn
346 Thr Asn Glu Ile Asn Val Val Asn Ala Val Asn Glu Val Asn Lys
361 Val Asn Asp Ser Asn Asn Ser Asn Asp Ala Asn Glu Gly Asn Asn
376 Ala Asn Tyr Ser Asn Asp Ser Ser Asn Thr Asn Asn Asn Thr Ser
391 Ser Ser Thr Asn Asn Ser Asn Asn Asn Thr Ser Cys Ser Ser Gln
406 Asn Thr Thr Thr Ser Ser Glu Asn Asn Asp Ser Leu Glu Asn Lys
421 Arg Asn Glu Glu Asp Glu Asp Glu Glu Asp Asp Gln Lys Asp Thr
436 Gln Lys Glu Lys Asn Asn Leu Glu Gln Glu Asp Met Ser Pro Tyr
451 Glu Asp Arg Asn Lys Asn Asp Glu Lys Asn Ile Asn Glu Gln Asp
466 Lys Phe His Leu Ser Asn Asp Leu Gly Lys Ile Tyr Asp Thr Tyr
481 Asn Gln Gly Asp Glu Val Val Val Ser Lys Asn Lys Asp Lys Leu
496 Glu Lys His Leu Asn Asp Tyr Lys Ser Tyr Tyr Tyr Leu Ser Lys
511 Ala Thr Leu Met Asp Lys Ile Gly Glu Ser Gln Asn Asn Asn Asn
526 Tyr Asn Val Cys Asn Ser Asn Glu Leu Gly Thr Asn Glu Ser Ile
541 Lys Thr Asn Ser Asp Gln Asn Asp Asn Val Lys Glu Lys Asn Asp
556 Ser Asn Ile Phe Met Lys Met Ile Ile Ile Ile Arg Leu Met Ile
571 Met Ile Ile Met Ile Met Ile Ile Ile Trp Tyr Leu Lys Ile Leu
586 Gln Asp Lys Ile Ile Trp Arg Asn Lys Lys Val Glu Lys Thr Ser
601 Asn Ile Leu Asn Asn Phe Asp Asn Asn Gly Asn Asp Asn Asp Asn
616 Asp Asn Asp Asp Asn Asn Asp Asn Asp Asn Asn Asn Asn Asn Asn
631 Met Asn Asn Gln Tyr Asn Tyr Gln Glu Asn Asn Ile Asn Thr Asn
646 Tyr Asn Ile Leu Tyr Thr Pro Ser Asn Cys Gln Ile Gln Asn Asn
661 Ser Tyr Met Asn Thr Asn Glu Met Tyr Gln Pro Leu Tyr Asn Thr
676 Tyr Pro Ser Asn Arg Ile Gln Glu Asn Ser Thr Ile Asn Asn Asn
691 Ile Ile Asn Asp Ser Pro Tyr Met Asn Asn Asp Asn Thr Thr Asn
706 Asn Thr Phe Ile Ser Gly Met Asn
```

Diese Aminosäuresequenz enthält 713 Aminosäurereste mit folgender Aminosäurezusammensetzung: Ala (19), Arg (6), Asn (219), Asp (37), Cys (3), Gln (15), Glu (65), Gly (10), His (5), Ile (34), Leu (26), Lys (48), Met (34), Phe (4), Pro (5), Ser (39), Thr (27), Trp (2), Tyr (23) und Val (92). Das berechnete Molekulargewicht dieses N-terminalen Teils des neuen Sporozoitenantigens beträgt 81'281 Da. Mögliche Glykosylierungsstellen befinden sich an den Positionen 32, 260, 308, 323, 329, 344, 362, 365, 377, 380, 387, 388, 394, 398, 399, 406, 414, 537, 554, 659, 684, 693, 702, 705 der Aminosäuresequenz (I).

Wesentliche Merkmale der Aminosäuresequenz (I) sind a) die Häufigkeit des Vorkommens von Asparaginresten in der Sequenz, sowie b) die in der N-terminalen Hälfte wiederholt vorkommende, drei Aminosäurereste umfassende Sequenz (NXY), in der N für Asparagin und X und Y für eine beliebige Aminosäure stehen. Bevorzugt ist der unmittelbar auf den Asparaginrest folgende Aminosäurerest X geladen, besonders bevorzugt sind die Aminosäurereste von Glutaminsäure und Lysin. Der Aminosäurerest Y an dritter Stelle ist bevorzugt ein hydrophober Aminosäurerest, besonders bevorzugt sind die Aminosäurereste von Valin und Methionin. In der Aminosäuresequenz (I) ist die Sequenz (NXY) etwa 103 mal wiederholt. Beispiele für besonders bevorzugte Sequenzen (NXY) sind AsnGluVal, AsnLysVal und AsnGluMet. Es ist für den Fachmann klar, dass die Sequenz $(NXY)_n$ auch die permutierten Sequenzen, d.h. auch $(YNX)_n$ sowie $(XYN)_n$ umfasst. Polypeptide mit zunehmender Anzahl n der repetierenden Sequenzen NXY, YNX bzw. XYN sind immunologisch kaum mehr unterscheidbar. Als Beispiele seien die Peptide $(AsnGluVal)_4$, $(ValAsnGlu)_4$ bzw. $(GluValAsn)_4$ erwähnt. Der Vergleich der Sequenz dieser Peptide, d.h.

AsnGluValAsnGluValAsnGluValAsnGluVal
GluValAsnGluValAsnGluValAsnGluValAsn
ValAsnGluValAsnGluValAsnGluValAsnGlu

zeigt klar, dass diese Peptide bis auf die endständigen Aminosäuren untereinander gleich sind.

Das Gen, das für das neue Sporozoitenantigen kodiert, enthält folgende Nukleotidsequenz (1), die für die N-terminale Aminosäuresequenz (I) kodiert:

```
             10         20         30         40         50         60
              |          |          |          |          |          |
   1 GAATTCCTCG TGATCATATG AGATATAGTT TTTATGTTGA GAATATTTGT TCAAAGAAGA
  61 TGCGTCACAA GAGAAGAAAC ATAAGCGCAT TTAATAAAAT TTTATATAAG AATTTTAAAT
 121 CAATCAAAAA ACAATTTACT ATGTTTCATC GTCCAAACAT GTTAAATTTA TCTTATGATT
 181 GGGATTCATT CCAAAAAGAT ATTACGGTAT TAGTAAACCA AATATTAACC TCTTACGAAT
 241 GGCATGGACA TGGAGCTTTT GAATCATTCG TTAAGTTATA TGGAGATTTA AGAAAAGATA
 301 TTTTAAGTCC TATACATCTA CTAAAAGTGT TGTGGAAAAT TTTAGAAATA TAGAAGAAGA
 361 CTTAGCTGAT TTAGATGAAG ATTCAACAGA AAATATTAAT GAACCTAATC ATTTAGATGG
 421 TCAAAATAAT AAAAACAATA GAAAAACTAA TAATGATAAT ACATTGAAAC AAAATCATCG
 481 AAAATCTAGG GGCACATCTG TACAAGGACG CAAAAATAAA ATAAATCGGG GATCAAAAGG
 541 CAAACATAAT TCTATAAATA TTCCAAAAGA TAGAAAGACG AACATAATGT CACAAATTAA
 601 TAAATTACTA TTTAATAAAA AAGATATTAA AATAAAATGT GAAGAAAGTA GTAGTTCAAA
 661 TTATGAAGAG GGAAATAGTT CGAGTAATGA AGAAAATAAT ATATCAACTG ATAAAAATAT
 721 TTGTAATACA AATAATAAAA ATGGAGTTTC ATTATATGAT AATTCAAAGG TTTATTTGAA
 781 AGGAGATTAT AAAATTTAGC AAACAGTTTC ATAAGTACAT ACACAAGAAC CTTTTAGATA
 841 ATGTCGATAA AACAGATAGA ACAATTAATA TTATATCAAA ATTCTTCGGT GGTGTGAATA
 901 AGTCCAATAA CGTGAATAAT ATTAATAGTG TAAATAAGGA GAATAACATG AATAAGGTGA
 961 ATGCTGTACA TAAGATAAAT GCTGTGGATA AGGTAAATGC TGTGAATAAG GTAAATTCTG
1021 TCAATAAGCT AAATGTTGTG AATAAGACGA ATGTTCTGAG TAAGTTGAAT GCTGTGTATA
1081 AGGTGAATTC TGTACATAAG ATGAATGCTG TGAATAAGGT AAATGCTGTA AATAAGGTGA
1141 ATGCTGTAAA TAAGGTAAAT GTCGTGAATA AGAAGGATAT TCTGAATAAG TTGAATGCTT
1201 TGTATAAGAT GAATGCTGTG TATAAAATGA ATGCTTTGAA TAAGGTGAGT GCTGTGAATA
1261 AGGTGAGTGC TGTGAATAAG GTGAGTGCTG TGAATAAGAT GGGTGCTGTA AATAGGGTGA
1321 ATGGAGTAAA CAAGGTGAAT GAGGTGAATG AGGTGAATGA GGTGAATGAA GTGAATATGG
1381 TGAATGAAGT AAATGAGTTA AATGAGGTGA ATAATGTCAA TGCAGTGAAT GAAGTGAATA
1441 GTGTGAACGA GGTTAATGAA ATGAATGAGG TGAATAAGGT GAATGAGCTA AATGAGGTGA
1501 ATGAAGTGAA TAATGTCAAT GAGGTGAATA ATGTGAATGT GATGAATAAT GTGAATGAGA
1561 TGAATAATAT GAATGAGATG AATAATGTCA ATGTAGTGAA TGAAGTGAAT AATGTCAATG
1621 AGGTGAATAA TGTGAATGAG ATGAATAATG TGAATGAGAT GAATAATATG AATGAGATGA
1681 ATAATGTCAA TGTAGTGAAT GAAGTGAATA ATGTAAATGA GATGAATAAT ACGAATGAGC
1741 TAAATGAGGT GAATGAAGTG AATAATGTGA ATGAGGTGAA TGATGTCAAT GTAGTGAACG
1801 AAGTGAATAA TGTAAATGAG ATGAATAATA TGAATGAGCT AAATGAGGTG AATGGGGTAA
1861 ATGAAGTGAA TAATACGAAT GAGATACATG AGATGAATAA TATAAATGAG GTGAATAATA
1921 CGAATGAGGT GAATAATACG AATGAGATAT ATGAGATGAA TAATATGAAT GATGTGAATA
1981 ATACGAATGA GATAAATGTG GTGAATGCGG TTAATGAAGT GAATAAGGTG AATGATTCAA
2041 ATAATTCAAA TGATGCAAAT GAAGGAAATA ACGCAAATTA TTCAAATGAT TCAAGCAATA
2101 CAAATAATAA CACATCAAGC AGCACAAATA ACTCAAATAA TAATACATCG TGTAGTTCAC
2161 AGAACACCAC AACTAGCAGC GAAAATAATG ATTCATTAGA AAATAAAAGA AATGAAGAAG
2221 ATGAAGATGA AGAAGACGAC CAAAAAGATA CACAAAAAGA AAAAAACAAT TTAGAACAGG
2281 AAGATATGAG TCCATACGAA GATAGAAATA AAAATGATGA AAAAAATATT AATGAACAAG
2341 ATAAATTTCA TTTATCAAAT GATTTGGGAA AAATATATGA TACATATAAC CAAGGAGATG
2401 AAGTTGTTGT ATCTAAGAAT AAGGACAAAT TAGAAAAGCA TTTGAATGAT TACAAGAGTT
2461 ATTATTATTT ATCTAAAGCA ACACTCATGG ACAAAATTGG AGAATCACAA AATAATAACA
2521 ACTATAATGT ATGTAATTCA AATGAACTTG GAACTAATGA ATCCATAAAG ACAAATTCTG
2581 ATCAGAATGA TAATGTAAAA GAAAAAAATG ATTCCAACAT ATTTATGAAA ATGATAATTA
2641 TAATTCGTCT TATGATAATG ATCATAATGA TAATGATAAT AATATGGTAT TTAAAGATTC
2701 TTCAAGACAA GATAATATGG AGAAACAAAA AAGTGGAGAA AACAAGCAAT ATTTTAAACA
2761 ATTTCGATAA TAATGGTAAT GATAATGATA ATGATAATGA TGATAATAAT GATAATGATA
2821 ATAATAATAA TAATAATATG AATAATCAAT ATAATTATCA AGAAAATAAT ATTAACACAA
2881 ATTATAACAT TTTGTACACT CCTTCTAATT GCCAAATCCA AAACAATTCA TATATGAATA
2941 CAAATGAAAT GTACCAACCA TTATATAATA CATATCCTTC AAATCGTATT CAAGAAAATT
3001 CTACTATAAA TAACAACATT ATTAATGATT CACCTTACAT GAATAACGAC AACACCACTA
3061 ATAACACCTT CATTTCTGGT ATGAATTC
```

Die vorliegende Erfindung betrifft das neue Plasmodium Sporozoitenantigen per se sowie Derivate davon, insbesondere Polypeptide, die in mindestens einem spezifischen Epitop mit dem Plasmodium Sporozoitenantigen mit der N-terminalen Aminosäuresequenz (I) übereinstimmen. Ein spezifisches Epitop ist definiert als eine immunogene Determinante auf einem Polypeptid, die sich durch eine spezifische molekulare Konfiguration einer Teilsequenz des Polypeptids ergibt. Bevorzugt sind die Polypeptide, die die repetierende Sequenz NXY bzw. deren Permutationen YNX bzw. XYN enthalten. Besonders bevorzugt sind daher Polypeptide, die die Sequenz $(NXY)_n$, $(YNX)_n$ bzw. $(XYN)_n$ enthalten, worin n eine Zahl zwischen 3 und 120 ist. Die Erfindung betrifft auch Polypeptide wie sie oben definiert sind und die zusätzlich noch kovalent mit einem weiteren Peptid am N-Terminus und/oder am C-Terminus verknüpft sind. Solche Fusionspolypeptide können durch die allgemeinen Formeln

A-B, B-C oder A-B-C

dargestellt werden, worin B ein Polypeptid ist, das in mindestens einem spezifischen Epitop mit dem Plasmodium Sporozoitenantigen mit der Aminosäuresequenz (I) übereinstimmt. Die zusätzlichen Peptide A und/oder C können im Prinzip beliebige Peptide sein, sind aber bevorzugt Affinitätspeptide oder T-Zellepitoppeptide. Unter einem Affinitätspeptid werden solche Peptide verstanden, die eine Aminosäuresequenz enthalten, die bevorzugt an ein Affinitätschromatographieträgermaterial bindet. Beispiele für solche Affinitätspeptide sind Peptide, die mindestens zwei, bevorzugt sechs Histidinreste enthalten. Solche Affinitätspeptide binden selektiv an Nitrilotriessigsäure-Nickelchelatharze (siehe z.B. europäische Patentanmeldung, Publ.-Nr. 253 303). Fusionspolypeptide, die solche Affinitätspeptide enthalten, können mittels Nitrilotriessigsäure-Nickelchelatharzen selektiv von den übrigen Polypeptiden abgetrennt werden (siehe z.B. die europäischen Patentanmeldungen mit den Publikationsnummern 282 042 und 309 746). Das Affinitätspeptid kann entweder mit dem C-Terminus oder dem N-Terminus des oben definierten Polypeptides B verknüpft sein, bevorzugt ist jedoch die Verknüpfung mit dem N-Terminus, z.B. dann, wenn das natürliche Stop-Codon des Plasmodium Sporozoitenantigens bei der Expression des erfindungsgemässen Polypeptids mitverwendet wird. Unter einem T-Zellepitoppeptid werden solche Peptide verstanden, die auf T-Zellen einwirken und dabei zur Kooperation von T- und B-Zellen bei der Immunantwort gegen das Malariaantigen beitragen. Besonders bevorzugt sind universelle T-Zellepitope wie sie z.B. in der europ. Patentanmeldung mit der Publikationsnummer 343 460 beschrieben sind.

Ein Beispiel für ein erfindungsgemässes Fusionspolypeptid der allgemeinen Formel

A-B-C

ist das Polypeptid mit der Aminosäuresequenz (II)

| | | | | | 5 | | | | | 10 | | | | | 15 |
|---|---|---|---|---|---|---|---|---|---|---|---|---|---|---|---|
| 1 | Met | Arg | Gly | Ser | His | His | His | His | His | His | Gly | Ser | Val | Asn | Ser |
| 16 | Val | Asn | Lys | Glu | Asn | Asn | Met | Asn | Lys | Val | Asn | Ala | Val | His | Lys |
| 31 | Ile | Asn | Ala | Val | Asp | Lys | Val | Asn | Ala | Val | Asn | Lys | Val | Asn | Ser |
| 46 | Val | Asn | Lys | Leu | Asn | Val | Val | Asn | Lys | Thr | Asn | Val | Leu | Ser | Lys |
| 61 | Leu | Asn | Ala | Val | Tyr | Lys | Val | Asn | Ser | Val | His | Lys | Met | Asn | Ala |
| 76 | Val | Asn | Lys | Val | Asn | Ala | Val | Asn | Lys | Val | Asn | Ala | Val | Asn | Lys |
| 91 | Val | Asn | Val | Val | Asn | Lys | Lys | Asp | Ile | Leu | Asn | Lys | Leu | Asn | Ala |
| 106 | Leu | Tyr | Lys | Met | Asn | Ala | Val | Tyr | Lys | Met | Asn | Ala | Leu | Asn | Lys |
| 121 | Val | Ser | Ala | Val | Asn | Lys | Val | Ser | Ala | Val | Asn | Lys | Val | Ser | Ala |
| 136 | Val | Asn | Lys | Met | Gly | Ala | Val | Asn | Arg | Val | Asn | Gly | Val | Asn | Lys |
| 151 | Val | Asn | Glu | Val | Asn | Glu | Val | Asn | Glu | Val | Asn | Glu | Val | Asn | Met |
| 166 | Val | Asn | Glu | Val | Asn | Glu | Leu | Asn | Glu | Val | Asn | Asn | Val | Asn | Ala |
| 181 | Val | Asn | Glu | Val | Asn | Ser | Val | Asn | Glu | Val | Asn | Glu | Met | Asn | Glu |
| 196 | Val | Asn | Lys | Val | Asn | Glu | Leu | Asn | Glu | Val | Asn | Glu | Val | Asn | Asn |
| 211 | Val | Asn | Glu | Val | Asn | Asn | Val | Asn | Val | Met | Asn | Asn | Val | Asn | Glu |
| 226 | Met | Asn | Asn | Met | Asn | Glu | Met | Asn | Asn | Val | Asn | Val | Val | Asn | Glu |
| 241 | Val | Asn | Asn | Val | Asn | Glu | Val | Asn | Asn | Val | Asn | Glu | Met | Asn | Asn |
| 256 | Val | Asn | Glu | Met | Asn | Asn | Met | Asn | Glu | Met | Asn | Asn | Val | Asn | Val |
| 271 | Val | Asn | Glu | Val | Asn | Asn | Val | Asn | Glu | Met | Asn | Asn | Thr | Asn | Glu |
| 286 | Leu | Asn | Glu | Val | Asn | Glu | Val | Asn | Asn | Val | Asn | Glu | Val | Asn | Asp |
| 301 | Val | Asn | Val | Val | Asn | Glu | Val | Asn | Asn | Val | Asn | Glu | Met | Asn | Asn |
| 316 | Met | Asn | Glu | Leu | Asn | Glu | Val | Asn | Gly | Val | Asn | Glu | Val | Asn | Asn |
| 331 | Thr | Asn | Glu | Ile | His | Glu | Met | Asn | Asn | Ile | Asn | Glu | Val | Asn | Asn |
| 346 | Thr | Asn | Glu | Val | Asn | Asn | Thr | Asn | Glu | Ile | Tyr | Glu | Met | Asn | Asn |
| 361 | Met | Asn | Asp | Val | Asn | Asn | Thr | Asn | Glu | Ile | Asn | Val | Val | Asn | Ala |
| 376 | Val | Asn | Glu | Val | Asn | Lys | Val | Asn | Asp | Ser | Asn | Asn | Ser | Asn | Asp |
| 391 | Ala | Asn | Glu | Gly | Asn | Asn | Ala | Asn | Tyr | Ser | Asn | Asp | Ser | Ser | Asn |
| 406 | Thr | Asn | Asn | Asn | Thr | Ser | Ser | Ser | Thr | Asn | Asn | Ser | Asn | Asn | Asn |
| 421 | Thr | Ser | Cys | Ser | Ser | Gln | Asn | Thr | Thr | Thr | Ser | Ser | Glu | Asn | Asn |
| 436 | Asp | Ser | Leu | Glu | Asn | Lys | Arg | Asn | Glu | Glu | Asp | Glu | Asp | Glu | Glu |
| 451 | Asp | Asp | Gln | Lys | Asp | Thr | Gln | Lys | Glu | Lys | Asn | Asn | Leu | Glu | Gln |
| 466 | Glu | Asp | Met | Ser | Pro | Tyr | Glu | Asp | Arg | Asn | Lys | Asn | Asp | Glu | Lys |
| 481 | Asn | Ile | Asn | Gly | Ile | Arg | Arg | Pro | Ala | Ala | Lys | Leu | Asn | | |

Dieses Polypeptid enthält 493 Aminosäurereste, wobei der Teil A die Aminosäurereste 1 bis 21, Teil B die Aminosäurereste 22 bis 483 und Teil C die Aminosäurereste 484 bis 493 umfasst. Das Polypeptid hat ein berechnetes Molekulargewicht von 55'239 Dalton. Teil A ist ein Affinitätspeptid mit sechs Histidinresten. Teil B entspricht dem N-terminalen Teil des Sporozoitenantigen der vorliegenden Erfindung, und enthält Aminosäurereste 1 bis 462 der Aminosäuresequenz (I). Teil C ist ein beliebiges Peptid das von einer Vektorsequenz kodiert wird.

Die Erfindung betrifft auch Polypeptide und Fusionspolypeptide, die durch Additionen, Deletionen oder Insertionen aus den oben gezeigten Aminosäuresequenzen hervorgegangen sind, vorausgesetzt, dass diese Polypeptide noch fähig sind, eine Immunantwort gegen das Circumsporozoitenstadium der Malariaparasiten auszulösen, vorzugsweise gegen das Sporozoitenantigen mit der N-terminalen Aminosäuresequenz (I) von P. falciparum. Die Erfindung betrifft ferner DNAs, die für ein erfindungsgemässes Polypeptid kodieren, sowie replizierbare mikrobielle Vektoren, die eine solche DNA enthalten, insbesondere Expressionsvektoren, d.h. replizierbare mikrobielle Vektoren, bei denen eine DNA, die für ein erfindungsgemässes Polypeptid kodiert, so an eine Expressionskontrollsequenz gebunden ist, dass das von der DNA kodierte Polypeptid in Mikroorganismen exprimiert werden kann. Ausserdem betrifft die vorliegende Erfindung Mikroorganismen, die einen solchen replizierbaren Vektor bzw. einen Expressionsvektor enthalten, sowie Verfahren zur Herstellung dieser Vektoren und Mikroorganismen. Im weiteren betrifft die vorliegende Erfindung Verfahren zur Herstellung der Polypeptide sowie deren Verwendung zur Immunisierung von Säugern gegen Malaria.

Da gewisse Substitutionen in der Aminosäuresequenz eines Polypeptids keinen Einfluss auf die räumliche Struktur bzw. die biologische Aktivität des Polypeptids haben, kann die Aminosäuresequenz der erfindungsgemässen Polypeptide von den oben gezeigten Aminosäuresequenzen abweichen. Beispiele solcher Aminosäuresubstitutionen sind Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu und vice versa (vgl. Doolittle, in "The Proteins", ed. Neurath, H, und Hill, R.L., Academic Press, New York [1979]).

Die erfindungsgemässen Polypeptide können kovalent an ein Trägermaterial gebunden oder daran adsorbiert sein. Geeignete Trägermaterialien sind natürliche oder synthetische Polymerverbindungen, wie z.B. Copolymere einer oder mehrerer Aminosäuren (z.B. Polylysin) oder Zucker (z.B. Polysaccharid). Weitere geeignete Trägermaterialien sind natürliche Polypeptide wie Hämocyanine (z.B. KLH = "keyhole limpet hemocyanin"), Serumproteine (z.B. Gammaglobulin, Serumalbumin) und Toxoide (z.B. Diphtherie- oder Tetanustoxoid). Weitere geeignete Trägermaterialien sind dem Fachmann bekannt.

Die kovalente Bindung der erfindungsgemässen Polypeptide an die Trägermaterialien kann auf bekannte Art und Weise erfolgen, z.B. direkt durch die Bildung einer Peptid- oder Esterbindung zwischen freien Carboxyl-, Amino- oder Hydroxylgruppen der Polypeptide und den entsprechenden Gruppen auf dem Trägermaterial, oder indirekt durch Verwendung von konventionellen, bifunktionellen Reagenzien wie z.B. m-Maleimidobenzoyl-N-hydroxysuccinimidester (MBS) oder Succinimidyl 4-(p-maleimidophenyl)butyrat (SMPB). Diese und weitere bifunktionellen Reagenzien sind käuflich erhältlich, z.B. von Pierce Chemical Company, Rockford, Illinois, U.S.A. Ferner können $C_{2-7}$-Dialkanale, wie z.B. Glutaraldehyd (Avrameas, Immunochem. 6, 43-52 [1969]), verwendet werden.

Das Trägermaterial, mit den daran gebundenen Polypeptiden, kann mittels bekannter Methoden (z.B. Dialyse oder Säulenchromatographie) von nichtgebundenen Polypeptiden und gegebenenfalls von den überschüssigen Reagentien abgetrennt werden.

Die Polypeptide der vorliegenden Erfindung insbesondere solche mit weniger als 50 Aminosäureresten können mittels konventioneller Methoden der Peptidsynthese, in flüssiger oder, vorzugsweise an fester Phase, gemäss der Methode von Merrifield (J. Am. Chem. Soc. 85, 2149-2154 [1963]), oder mittels anderer gleichwertiger Methoden des Standes der Technik, hergestellt werden.

Die Festphasensynthese beginnt mit der C-terminalen Aminosäure des zu synthetisierenden Peptids, die in geschützter Form an ein passendes Trägermaterial gekoppelt wird. Das Ausgangsmaterial kann durch Bindung einer aminogruppengeschützten Aminosäure über eine Benzylesterbrücke an chlormethyliertes oder ein hydroxymethyliertes Trägermaterial oder durch Amidbindung an ein Benzhydrylamin (BHA)-, ein Methylbenzhydrylamin (MBHA)- oder ein Benzyloxybenzylalkohol-Trägermaterial hergestellt werden. Diese Trägermaterialien sind käuflich erhältlich und ihre Bereitstellung und Verwendung sind wohlbekannt.

Allgemeine Methoden zum Schützen und Entfernen der Schutzgruppen von Aminosäuren, die in dieser Erfindung verwendet werden können, sind beschrieben in "The Peptides", Vol. 2 (herausgegeben von E. Gross und J. Meienhofer, Academic Press, New York, 1-284 [1979]). Schutzgruppen umfassen z.B. die 9-Fluorenylmethyloxycarbonyl (Fmoc), die tertiäre Butyloxycarbonyl (Boc)-, die Benzyl (Bzl)-, die t-Butyl (But)-, die 2-Chlorbenzyloxycarbonyl (2Cl-Z)-, die Dichlorbenzyl (Dcb)- und die 3,4-Dimethylbenzyl-(Dmb)-gruppe.

Nach Entfernen der $\alpha$-Aminoschutzgruppe der an den Träger gebundenen C-terminalen Aminosäure werden die geschützten Aminosäuren in der gewünschten Reihenfolge schrittweise angekoppelt. Ein vollständiges Polypeptid kann so synthetisiert werden. Als Alternative dazu können kleine Peptide aufgebaut werden, die dann zum gewünschten Polypeptid zusammengefügt werden. Geeignete Kopplungsreagenzien gehören zum Stand der Technik, wobei Dicyclohexylcarbodiimid (DCC) besonders geeignet ist.

Jede(s) geschützte Aminosäure oder Peptid wird im Ueberschuss in das Festphasensynthesereaktionsgefäss gegeben und die Kopplungsreaktion kann in Dimethylformamid (DMF) oder Methylenchlorid ($CH_2Cl_2$) oder einer Mischung aus beiden durchgeführt werden. In Fällen von unvollständiger Kopplung wird die Kopplungsreaktion wiederholt bevor die N-terminale $\alpha$-Aminoschutzgruppe zwecks Kopplung der nächsten Aminosäure entfernt wird. Die Ausbeute jedes Kopplungsschrittes kann verfolgt werden und zwar bevorzugt nach der Ninhydrinmethode. Die Kopplungsreaktionen und die Waschschritte können automatisiert durchgeführt werden.

Die Abspaltung des Peptids vom Trägermaterial kann durch in der Peptidchemie wohlbekannte Methoden erreicht werden, z.B. durch Umsetzung mit Fluorwasserstoff (HF) in Gegenwart von p-Kresol und Dimethylsulfid während 1 Stunde bei 0 °C, gefolgt möglicherweise von einer zweiten Umsetzung mit HF in Gegenwart von p-Kresol während 2 Stunden bei 0 °C. Die Abspaltung der Peptide von chlormethylierten oder hydroxymethylierten Trägermaterialien ergibt Peptide mit freiem C-Terminus; die Abspaltung der Peptide von Benzhydrylamin- oder Methylbenzhydrylamin-Trägern ergibt Peptide mit amidiertem C-Terminus.

Andererseits können die Polypeptide der vorliegenden Erfindung auch unter Verwendung der Methoden der DNA-Rekombinationstechnik (Maniatis et al. in "Molecular Cloning - A Laboratory Manual", Cold Spring Harbor Laboratory [1982]) hergestellt werden. z.B. kann ein Stück DNA d.h. DNA-Fragment, das für ein solches Polypeptid kodiert, mittels konventioneller chemischer Methoden synthetisiert werden, z.B. mittels der Phosphotriestermethode wie sie von Narang et al. in Meth. Enzymol. 68, 90-108 [1979] beschrieben ist oder mittels der Phosphodiestermethode (Brown et al., Meth. Enzymol. 68, 109-151 [1979]). Bei beiden Methoden werden zuerst längere Oligonukleotid synthetisiert, die in vorgegebener Art und Weise aneinander gehängt werden. Die Nukleotidsequenz des DNA-Fragmentes kann identisch sein zu derjenigen Nukleotidsequenz, die das natürliche Polypeptid im Plasmodium Parasiten kodiert. Da der genetische Code degeneriert ist, besteht andererseits die Möglichkeit, dass eine teilweise oder vollständig unterschiedliche Nukleotidsequenz das gleiche Polypeptid kodiert. Gegebenenfalls können für die Nukleotidsequenz solche Codons gewählt werden, die auch vom Wirtsorganismus, der zur Expression des Polypeptids verwendet wird, bevorzugt verwendet werden (Grosjean et al., Gene 18, 199-209 [1982]). Dabei muss aber darauf geachtet werden, dass das so erhaltene DNA-Fragment keine Teilsequenzen enthält, die die Konstruktion des Expressionsvektors, z.B. durch Einführung einer unerwünschten Restriktionsenzymschnittstelle, erschweren oder die die Expression des Polypeptids behindern.

Das DNA-Fragment das für das erfindungsgemässe Plasmodium Sporozoitenantigen bzw. für die Teilsequenz B des erfindungsgemässen Fusionspolypeptids kodiert kann auch erhalten werden, indem genomische DNA eines Plasmodiumstammes mit einer oder mehreren geeigneten Restriktionsendonukleasen, z.B. EcoRI, gespalten wird. Fragmente mit einer Länge von 1,5 bis 6 x $10^3$ Basenpaaren werden isoliert und in einen geeigneten Vektor, z.B. in den λ-Phagenvektor gt11 eingebaut. Der Vektor gt11 ist von Young et al., Proc. Natl. Acad. Sci. USA 80, 1194-1198 [1983] beschrieben und bei der American Type Culture Collection (ATCC), 12301 Parklawn Drive, Rockville, Maryland, USA oder von anderen Stellen erhältlich. Die rekombinierte Phagen-DNA kann in vitro in Phagenproteinhüllen gepackt werden. Die so erhaltenen infektiösen Phagen werden in geeignete Wirtszellen, z.B. in E. coli Y1088 enthaltend das Plasmid pMC9 (erhältlich bei ATCC) eingeführt. Aus etwa 100'000 rekombinierten Phagen werden diejenigen Phagen gesucht, die mit einer geeigneten Probe reagieren. Solche geeigneten Proben sind Oligonukleotide, die einer Teilsequenz der genomischen DNA entsprechen, die für ein erfindungsgemässes Polypeptid kodiert oder Antikörper die von λ-gt11 Phagen produziertes Sporozoitenantigen erkennen. Die Art und Weise wie diese Proben ausgewählt und verwendet werden ist dem Fachmann bekannt. Phagen, die das gewünschte DNA-Fragment enthalten, werden vermehrt und die DNA isoliert. Das DNA-Fragment kann anschliessend in einen geeigneten replizierbaren mikrobiellen Vektor eingebaut werden, vorzugsweise in einen Expressionsvektor, der die notwendigen Expressionssignale liefert und der ggf. für die Teilsequenzen A und/oder C der obengenannten Fusionspolypeptide kodiert. Ein bevorzugter Expressionsvektor ist der Vektor pDS56/RBSII,6xHis, der in den Beispielen beschrieben ist. Die Polypeptide der vorliegenden Erfindung können, nach entsprechender Anpassung der Nukleotidsequenz, auch in anderen geeigneten Expressionsvektoren hergestellt werden. Beispiele solcher Expressionsvektoren sind in der Europäischen Patentanmeldung, Publikations-Nr. 186 069, die am 2. Juli 1986 veröffentlicht worden ist, beschrieben. Weitere Expressionsvektoren sind dem Fachmann bekannt.

Der Expressionsvektor, der ein DNA-Fragment mit der DNA Sequenz, die für ein erfindungsgemässes Polypeptid kodiert, enthält, wird anschliessend in einem geeigneten Wirtorganismus eingeführt. Geeignete Wirtsorganismen sind Mikroorganismen, z.B. Hefezellen oder Bakterienzellen die fähig sind, das von Expressionsvektoren kodierte Polypeptid zu exprimieren. Bevorzugter Wirtsorganismus ist E.coli SG13009. Weitere geeignete Wirtsorganismen sind E. coli M15 (von Villarejo et al. in J. Bacteriol. 120, 466-474 [1974] als DZ291 beschrieben), E.coli 294, E.coli RR1 und E.coli W3110, die alle bei ATCC, DSM oder von anderen Stellen erhältlich sind.

Die Art und Weise wie die Expression der erfindungsgemässen Polypeptide erfolgt, ist abhängig vom verwendeten Expressionsvektor und vom Wirtsorganismus. Ueblicherweise werden die Wirtsorganismen, die den Expressionsvektor enthalten, unter Bedingungen, die für das Wachstum der Wirtsorganismen optimal sind, vermehrt. Gegen Ende des exponentiellen Wachstums, wenn die Zunahme der Zellzahl pro Zeiteinheit abnimmt, wird die Expression des Polypeptids der vorliegenden Erfindung induziert, d.h. die das Polypeptid kodierende DNA transkribiert und die transkribrierte mRNA in Protein translatiert. Die Induktion kann durch Zugabe eines Induktors oder eines Derepressors zum Wachstumsmedium oder durch Veränderung eines physikalischen Parameters z.B. einer Temperaturänderung erfolgen. In dem in der vorliegenden Erfindung verwendeten Expressionsvektor, wird die Expression durch den lac-Repressor kontrolliert, der an eine Kontrollsequenz bindet. Durch Zugabe von Isopropyl-β-D-thiogalactopyranosid (IPTG) wird der Repressor entfernt und dadurch die Synthese des Polypeptids induziert. Andere Induktionssysteme sind dem Fachmann bekannt.

Das Translationsstartsignal AUG, dem auf dem Niveau der DNA das Codon ATG entspricht, bewirkt, dass alle Polypeptide in einem prokaryotischen Wirtsorganismus mit einem Methioninrest am N-Terminus synthetisiert werden. In gewissen Expressionssystemen wird dieser N-terminale Methioninrest abgespalten.

Es hat sich aber gezeigt, dass die An- oder Abwesenheit des N-terminalen Methionins, die biologische Aktivität eines Polypeptids kaum beeinflusst (vergl. Winnacker, in "Gene und Klone", S. 255, Verlag Chemie, Weinheim, BRD [1985]). In Fällen in denen das N-terminale Methionin stört, kann es auch mittels einer für das N-terminale Methionin spezifischen Peptidase abgespalten werden. Miller et al. (Proc. Natl. Acad. Sci. U.S.A. 84, 2718-2722 [1987]) haben die Isolation einer solchen Peptidase aus Salmonella typhimurium beschrieben. Die vorliegende Erfindung betrifft deshalb Polypeptide mit oder ohne N-terminalen Methioninrest.

Die in den Wirtsorganismen produzierten Polypeptide können durch spezielle Transportmechanismen aus der Zelle sekretiert, oder durch Aufbrechen der Zelle isoliert werden. Das Aufbrechen der Zelle kann durch mechanische (Charm et al., Meth. Enzymol. 22, 476-556 [1971]), enzymatische (Lysozymbehandlung) oder chemische (Detergenzbehandlung, Harnstoff- oder Guanidin•HCl-Behandlung, etc.) Einwirkungen erfolgen, oder durch eine Kombination dieser Einwirkungen.

Die erfindungsgemässen Polypeptide können danach mittels bekannter Methoden gereinigt werden, wie zum Beispiel mittels Zentrifugation bei unterschiedlichen Geschwindigkeiten, mittels Präzipitation mit Ammoniumsulfat, mittels Dialyse (bei Normaldruck oder bei reduziertem Druck), mittels präparativer Isoelektrofokussierung, mittels präparativer Gelelektrophorese oder mittels verschiedener chromatographischer Methoden wie Gelfiltration, Hochleistungs-Flüssigchromatographie (HPLC), Ionenaustauschchromatographie, Umkehrphasenchromatographie und Affinitätschromatographie (z.B. an Sepharose Blau CL-6B, an trägergebundenen, gegen das Polypeptid gerichteten monoklonalen Antikörpern oder an Metallchelatharzen).

Die Polypeptide der vorliegenden Erfindung können in Form von Multimeren, insbesondere in Form von Dimeren vorliegen wie sie in Fig. 8A schematisch dargestellt sind. Multimere können auch entstehen, wenn Polypeptide in prokaryotischen Wirtsorganismen hergestellt werden, insbesondere durch die Bildung von Disulfidbrücken zwischen Cysteinresten.

Die vorliegende Erfindung betrifft auch immunogene Kompositionen, die ein Polypeptid gemäss der vorliegenden Erfindung, sowie ein geeignetes Adjuvants, enthalten. Geeignete Adjuvantien für die Anwendung an Mensch und Tier sind dem Fachmann bekannt (Warren et al., Ann. Rev. Immunol. 4, 369-388 [1986]; Morein, Nature 332, 287-288 [1988]; Klausner, BIO/TECHNOLOGY 6, 773-777 [1988] und Bromford, Parasitology Today 5, 41-46 [1989]). Die erfindungsgemässen Polypeptide und immunogen Kompositionen können als Lyophilisate zur Rekonstitution mit sterilem Wasser oder einer Salzlösung, vorzugsweise einer Kochsalzlösung, vorliegen.

Durch Einführung der erfindungsgemässen Polypeptide und immunogenen Kompositionen in Säuger wird deren Immunsystem aktiviert und Antikörper gegen das erfindungsgemässe Plasmodium Sporozoitenantigen gebildet. Diese Antikörper können aus dem Serum isoliert werden. Die vorliegende Erfindung betrifft auch solche Antikörper. Die erfindungsgemässen Antikörper reagieren mit Malariaparasiten und können deshalb für die passive Immunisierung oder für diagnostische Zwecke verwendet werden. Antikörper gegen die erfindungsgemässen Polypeptide können in Affen, Kaninchen, Pferden, Ziegen, Meerschweinchen, Ratten, Mäusen, Kühen, Schafen etc., aber auch in Menschen hergestellt werden. Je nach Bedürfnis können das Antiserum oder die gereinigten Antikörper verwendet werden. Die Antikörper können auf bekannte Art und Weise, z.B. durch Fällung mit Ammoniumsulfat, gereinigt werden. Es ist auch möglich, monoklonale Antikörper, die gegen das Polypeptid der vorliegenden Erfindung gerichtet sind, gemäss der von Köhler et al. (Nature, 256, 495-497 [1975]) entwickelten Methode, herzustellen. Polyklonale oder monoklonale Antikörper können auch zur affinitäts-chromatographischen Reinigung der Polypeptide der vorliegenden Erfindung oder deren natürlichen Aequivalenten verwendet werden.

Die erfindungsgemässen Polypeptide und die immunogenen Kompositionen können zur Immunisierung von Säugern gegen Malaria verwendet werden. Die Art der Verabreichung, die Dosierung, sowie die Anzahl der Injektionen können vom Fachmann auf bekannte Art und Weise optimiert werden. Typischerweise werden über einen längeren Zeitraum mehrere Injektionen verabreicht, um einen hohen Titer an Antikörpern gegen den Malariaparasiten, d.h. gegen das Plasmodium Sporozoitenantigen der vorliegenden Erfindung, zu erhalten.

Die Figuren und das nachfolgende ausführliche Beispiel tragen zum besseren Verständnis der vorliegenden Erfindung bei. Es soll aber nicht der Eindruck erweckt werden, dass die Erfindung auf den Gegenstand des Beispiels oder der Figuren beschränkt ist.

## Legende zu den Figuren

Fig. 1 Aminosäuresequenz des N-terminalen Teils des P. falciparum Sporozoitenantigens = Aminosäuresequenz (I)

Fig. 2 Nukleotidsequenz des P. falciparum Gens das für das Sporozoitenantigen kodiert = Nukleotidsequenz (1)

Fig. 3 Aminosäuresequenz des Fusionsproteins mit der Aminosäuresequenz (II)

Fig. 4 Partielle Restriktionsenzymkarte der Vektoren NXY, NXY-L, NXY-H, M13-NXY und pDS-NXY. Der Vektor NXY enthält die Nukleotide 1-3088 der Nukleotidsequenz (1) im Lambdaphagen gt11. Der schwarze Balken definiert die kodierende Region, die vermutlich mit dem ATG Startcodon an Position 948-950 der Nukleotidsequenz (1) beginnt. Aus dem Vektor NXY wurde ein 1 kb EcoRI-Fragment (Nukleotide 1-1084) bzw. ein 2 kb EcoRI-Fragment (Nukleotide 1085-3088) isoliert und in den Vektor M13 mp18 integriert. Dies führte zu den Vektoren NXY-L enthaltend das 1 kb EcoRI-Fragment bzw. NXY-H enthaltend das 2 kb EcoRI-Fragment aus dem Vektor NXY. Der Vektor M13-NXY entspricht dem Vektor M13 mp18 enthaltend die Nukleotide 1-3088 der Nukleotidsequenz (1). Dieser Vektor enthält somit die gleiche DNA wie NXY. Der Vektor pDS-NXY enthält dar 1413 bp AseI Fragment (Nukleotide 922-2332 der Nukleotidsequenz (1)) aus M13-NXY, das nach Auffüllen der der überlappenden Enden mit Klenow-Polymerase mit BamHI Verbindungsstücken (10-mer) versehen und in pDS56/RBSII,6xHis kloniert wurde. Die korrekte Orientierung zum Promoter wurde über Restriktionsanalyse ermittelt. E. coli Zellen, die mit pDS-NXY transformiert sind produzieren nach Induktion das rekombinierte Plasmodium Sporozoitenantigen der allgemeinen Formel A-B-C mit der in Fig. 3 gezeigten Sequenz. Die Aminosäuresequenz dieses Proteins wird von den Nukleotiden 922 bis 2332 der Nukleotidsequenz (1) kodiert.

Fig. 5 Graphische Darstellung der Verteilung von positiv (Ordinate obere Hälfte) und negativ (Ordinate untere Hälfte) geladenen Aminosäuren in der Aminosäuresequenz (I). Die Numerierung der Aminosäuren (Abszisse) entspricht derjenigen in Fig. 1. Die Graphik wurde mit dem PC/Gene Programm "NOVOTNY" (Genofit SA, Genf, Schweiz) erstellt.

Fig. 6 Graphische Darstellung der hydrophoben Proteindomänen in der Aminosäuresequenz (I) berechnet nach Kyte et al., J. Mol. Biol. 157, 105-132 (1982). Werte über dem -5 Index (Ordinate) weisen auf hydrophobe Domänen hin. Die Numerierung der Aminosäuren (Abszisse) entspricht derjenigen in Fig. 1.

Fig. 7 Potentielle B-Zellepitope in der Aminosäuresequenz (I). Werte über dem Null-Index (Ordinate) bedeuten das Vorhandensein von möglichen B-Zellepitopen. Die Berechnung erfolgte nach Hopp et al., Proc. Natl. Acad. Sci. 78, 3824-3828 (1981). Die Numerierung der Aminosäuren (Abszisse) entspricht derjenigen in Fig. 1.

Fig. 8 (A) Modell zur möglichen Dimer-Bildung von zwei erfindungsgemässen Molekülen des Sporozoitenantigens mittels intermolekularer Wechselwirkung zwischen den positiv und negativ geladenen Bereichen in der N-terminalen Aminosäuresequenz des erfindungsgemässen Plasmodium Sporozoitenantigens (s. auch Fig. 5). (B) Zeigt die Bereiche mit den unterschiedlichen Eigenschaften in der Aminosäuresequenz (I) gemäss den Computerberechnungen (siehe Fig. 5 bis 7). + = positiv geladene Domäne; - = negativ geladene Domäne; glyc = potentielle N-Glycosylierungsregion; ag = Antigenbereich (d.h. erhöhte Wahrscheinlichkeit für das Vorhandensein von B-Zellepitopen; tm = mögliche Transmembransequenz.

Fig. 9

A) Analyse von SspI verdauter, genomischer DNA von 9 verschiedenen P. falciparum Isolaten (Gentz et al., EMBO J. 7, 225-230 [1988]). Folgende Isolate wurden getestet: Spur 1 = T9/96,2; Spur 2 = #13; Spur 3 = CPG-1; Spur 4 = 542; Spur 5 = K1; Spur 6 = MAD20; Spur 7 = RO53; Spur 8 = T9/94; Spur 9 = RO-59.

B) Analyse von DraI- (Spuren 1 und 3) bzw. HindIII- (Spuren 2 und 3) verdauter genomischer DNA von zwei verschiedenen Plasmodium Spezies (P. falciparum Spuren 1 und 2; P. berghei Spuren 3 und 4).

Fig. 10 Nukleotidsequenz des Plasmides pDS56/RBSII,6xHis.

Die in der vorliegenden Anmeldung erwähnten Abkürzungen, Puffer und Medien, sowie die im Beispiel verwendeten Methoden 1 bis 15 entsprechen denjenigen, die in der Europäischen Patentanmeldung, Publikationsnummer 309 746 auf Seite 13 bis 20 beschriebenen sind.

## Beispiel

Isolation eines P.falciparum Sporozoitengens aus einer genomischen Expressions-Genbank unter Verwendung von Antikörpern

Herstellung eines Immunserums gegen P. falciparum Sporozoiten

Sporozoiten des P.falciparum Isolates NF54 wurden nach üblichen Methoden aus infizierten Anopheles stephensis Mücken isoliert. Ein Kaninchen wurde in 2-wöchigen Abständen mit je $10^6$ dieser Sporozoiten in komplettem Freund's Adjuvans immunisiert. Das Immunserum wurde auf übliche Art und Weise gewonnen.

Konstruktion der Expressionsgenbank von P.falciparum

P.falciparum-Zellen (K1-Isolat) wurden mittels üblicher Methoden (Trager et al., Science 193, 673-675 [1976]) in 10 Kulturschalen gezüchtet und anschliessend in Kulturmedium enthaltend 0,1% Saponin gewaschen. Die gewaschenen Parasiten wurden in 2 ml 10 mM EDTA [pH 8.0] 0,5% (w/v) SDS resuspendiert. Nach der Zugabe von 50 mg Proteinase K (Merck) wurde das Gemisch während 10 Minuten bei 65° C inkubiert und anschliessend mit 2 ml Phenol (gesättigt mit 1 M Tris/HCl [pH 8,0]) versetzt. Die Phasen wurden durch Schütteln vermischt und durch Zentrifugation (10 Minuten bei 6'000 RPM, 20° C) wieder getrennt. Die Phenolextraktion wurde zweimal wiederholt (es sollte keine Interphase mehr sichtbar sein). Die DNA in der wässrigen Phase wurde gemäss Methode 1 gefällt, mit Aethanol gewaschen und getrocknet. Die DNA wurde in 2 ml Wasser gelöst und mechanisch geschert, d.h. 80-mal durch eine Spritze mit einer 0,5 x 16 mm Nadel gedrückt. Danach wurden 0,2 Volumen 5 x EcoRI Methylasepuffer (50 mM Tris/HCl [pH 7,5], 0,25 M NaCl, 50 mM EDTA, 25 mM β-Mercaptoäthanol, 0,4 mM S-Adenosylmethionin) zugegeben. 10 μg DNA wurden mit 50 Einheiten EcoRI Methylase (New England Biolabs Beverly, Massachusetts, USA) während 30 Minuten bei 37° C methyliert. Die DNA wurde wie oben beschrieben einmal mit Phenol extrahiert und gemäss Methode 1 gefällt. Die DNA wurde in 200 μl T4-Polymerasepuffer gelöst und nach Zugabe von 5 μl 5 mM dATP, dCTP, dGTP und dTTP, sowie 10 Einheiten T4 Polymerase während 30 Minuten bei 37° C inkubiert. Die DNA wurde erneut mit Phenol extrahiert und gemäss Methode 1 gefällt. Die DNA wurde in 50 μl T4-DNA Ligasepuffer gelöst und nach Zugabe von 0,01 $OD_{260}$-Einheiten phosphorylierten EcoRI-Oligonukleotidadaptoren (New England Biolabs) und 2 μl T4-DNA-Ligase (12 Weiss-Einheiten) über Nacht bei 14° C ligiert. Die DNA wurde gemäss Methode 1 gefällt, in 20 μl 1 x DNA-Gelladepuffer gelöst und auf einem 0,8% (w/v) Agarosegel aufgetrennt (Methode 2). DNA Fragmente mit einer Länge von 2 bis 6 kb (1 kb = 1'000 Nukleotide) wurden gemäss Methode 3 isoliert. Die erhaltene DNA wurde in 50 μl Wasser gelöst und nach Zugabe von 6 μl 10 x Ligase Puffer, 2 μl dephosphorylierten Lambda-Armen (Promega Biotech., Madison WI, U.S.A.) und 6 Weiss-Einheiten T4-DNA-Ligase übernacht bei 14° C ligiert. Die DNA wurde gefällt (Methode 1) und in 5 μl Wasser gelöst. Nach Zugabe von 20 μl "Packaging Extract" (Genofit S.A., Genf, Schweiz) wurde die DNA während 2 Stunden bei 20° C nach Vorschrift des Lieferanten in Lambda-Phagenpartikel gepackt. Nach der Zugabe von 500 μl SM-Puffer sowie 50 μl Chloroform war die Genbank bereit für den Antikörpertest.

Antikörpertest der Genbank

E.coli Y1090 wurde in 3 ml LB-Medium enthaltend 40 μg/ml Ampicillin über Nacht bei 37° C im Schüttelbad inkubiert. Am anderen Morgen wurden die Zellen sedimentiert (10 Minuten bei 7'000 x g, 20° C) und in 1 ml SM-Puffer resuspendiert. Zu dieser Zellsuspension wurden $10^6$ infektiöse Phagenpartikel der Genbank zugegeben und während 30 Minuten bei Raumtemperatur inkubiert. Dann wurden 60 ml auf 42° C temperierte 0,8% (w/v) Agarlösung in LB-Medium zugegeben und gut vermischt. Der Weichagar mit den infizierten Zellen wurde auf 6 LB-Agarplatten (Durchmesser 135 mm) enthaltend 40 μg/ml Ampicillin verteilt und während 5 Stunden bei 42° C inkubiert. Auf jede Schale wurde ein, in 100 mM IPTG-Lösung getauchter und getrockneter, Nitrocellulosefilter gelegt und übernacht bei 37° C inkubiert. Am nächsten Tag wurde die Lage des Filters bezüglich der Schale markiert und der markierte Filter in 1 x TBS aufbewahrt. Ein neuer, in 100 mM IPTG-Lösung behandelter, Nitrocellulose Filter wurde auf die Platte gelegt, markiert und während 4 Stunden bei 37° C auf den Platten inkubiert. Beide Filtersätze wurden während 10 Minuten in 1 x TBS geschüttelt, dann 20 Minuten in 1 x TBS, 20% FCS (fötales Kälberserum) inkubiert. Das sporozoitenspezifische Kaninchenantiserum wurde 1:1000 mit 1 x TBS/20% FCS verdünnt und beide Filtersätze während einer Stunde bei Raumtemperatur in einem Schüttelbad inkubiert. Die Filter wurden nun dreimal während je zehn Minuten in 1 x TBS, 0,1% Triton®-X-100 in einem Schüttelbad gewaschen, gefolgt von einer einstündigen Inkubation mit 5 μCi [$^{125}$J]-Protein A (Amersham, Aylesbury, GB; Katalog Nr. 1M.144) in 1 x TBS, 0,1% proteasefreiem Rinderserumalbumin. Die Filter wurden nochmals wie oben gewaschen und dann bei Raumtemperatur getrocknet. Die Filter wurden gegen Kodak XAR-Röntgenfilm über Nacht exponiert. Plaques die auf beiden Platten positiv reagierten wurden mit Hilfe der Markierungen auf dem Film identifiziert und anhand der Markierung von den Petrischalen gepickt. Die Phagenlösung wurde nochmals in verschiedenen Verdünnungen gemäss Methode 4 in Softagar ausplattiert und einzelne positive Plaques nochmals wie oben beschrieben identifiziert. Ein positiver Plaque, nachfolgend NXY genannt, wurde gepickt, die Lambdaphagen gemäss Methode 5 vermehrt und die DNA isoliert.

10 μg NXY DNA wurden in 490 μl T4-Polymerasepuffer gelöst und mit 50 Einheiten EcoRI während einer Stunde bei 37° C verdaut. Die DNA wurde gefällt (Methode 1) und auf einem 0,8% (w/v) Agarosegel aufgetrennt (Methode 2). Als Kontrolle wurden 10 μg gt11 DNA mit EcoRI verdaut und analysiert. Zwei EcoRI Fragmente (2,0 und 1,0 kb) waren nur in der Spur mit der NXY DNA vorhanden. Die EcoRI Fragmente wurde isoliert (Methode 3) und in 50 μl Wasser gelöst. Zur Klonierung wurden 50 ng EcoRI geschnittene, dephosphorylierte M13 mp18 DNA (Pharmacia Uppsala, SE; Methode 6) mit je 10 μl der gelösten EcoRI Fragmente aus der NXY DNA gemischt, 2 μl 10 x Ligase Puffer, 6 μl Wasser und 6 Weiss-Einheiten T4-DNA Ligase zugegeben und die DNA's während einer Stunde bei Raumtemperatur ligiert. Kompetente TG-1 E.coli Zellen (Amersham) wurden mit der ligierten DNA transformiert (Methode 7). Von jedem Ansatz wurden zwei Plaques isoliert, amplifiziert und genügend DNA für die Bestimmung der Sequenz isoliert (Methode 8). Die DNA-Sequenz wurde gemäss der Methode 9 bestimmt. Die verwendeten M13 mp18 Klone mit den EcoRI Fragmenten wurden mit NXY-L (1 kb Fragment) bzw. NXY-H (2,0 kb Fragment) bezeichnet.

Einführung von Deletionen zur Sequenzbestimmung

Je 1 μg NXY-L und NXY-H DNA wurden mit BamHI und PstI komplett verdaut. Nach einer Stunde wurden die DNA's gefällt (Methode 1) und das Pellet wurde in 100 μl 66 mM Tris/HCl, [pH 8,0], 6,6 mM $MgCl_2$ gelöst. Nach Zugabe von 10 Einheiten Exonuclease III wurde das Gemisch bei 37° C inkubiert. Nach jeweils 1, 3 und 6 Minuten wurden 30 μl entnommen und mit 3 μl 0,5 M EDTA versetzt. Die Proben wurden nach Methode 1 gefällt. Die DNAs wurden in 50 μl S1-Nuklease-Puffer (0,3 M Kaliumacetat, 10 mM Zinksulfat, 5% (v/v) Glycerin) gelöst. Nach Zugabe von 10 Einheiten S1-Nuklease wurde das Gemisch während 30 Minuten bei Raumtemperatur inkubiert. Die Proben werden je einmal mit Phenol und Aether extrahiert und dann nach Methode 1 gefällt. Die DNAs wurden in HIN-Puffer gelöst und mit 5 Einheiten Klenow-Polymerase während 2 Minuten bei 37° C inkubiert. Nach Zugabe von 1 μl 0,25 mM dATP, dCTP, dTTP und dGTP wurde das Gemisch nochmals während 2 Minuten inkubiert. Nach Zugabe von 5 μl 10x Ligase-Puffer und 400 Einheiten T4-DNA Ligase wurde über Nacht bei Raumtemperatur ligiert. Anschliessend wurden E. coli Zellen gemäss Methode 7 mit der DNA transformiert. Die Sequenzanalyse der DNA erfolgte gemäss Methode 8 und 9.

Analyse der NXY Proteinsequenz

Die von der Nukleinsäuresequenz (1; Fig. 2) abgeleitete Proteinsequenz (I; Fig. 1) wurde hinsichtlich Ladungsverteilung (Fig. 5), Hydrophobizität (Fig. 6), sowie das Vorhandensein möglicher B-Zellepitope (Fig. 7) analysiert. Danach gibt es in der Aminosäuresequenz (I), beginnend am N-Terminus eine Anhäufung von positiven Ladungen, gefolgt von einer Anhäufung von negativen Ladungen. Danach folgen Aminosäurereste, die N-glykosyliert werden können (Fig. 8B) gefolgt von einer Region, die potententielle immunogene B-Zellepitope enthält. Im C-terminalen Teil der Aminosäuresequenz (I) befindet sich eine typische Transmembransequenz (Fig. 6 und 8B), die zur Verankerung des Proteins in der Sporozoitenmembran dienen könnte. Gemäss dieser Analyse der Aminosäuresequenz (I) scheint es möglich, dass die geladenen Regionen inklusive der immunogenen Domäne auf der Sporozoiten-Oberfläche und damit dem Immunsystem exponiert sind. Die Erkennung des erfindungsgemässen Sporozoitenantigens durch Humanseren von Malaria exponierten Individuen spricht für diese Hypothese (s. unten). Figure 8A zeigt wie zwei erfindungsgemässe Polypeptide mittels der geladenen N-Termini Dimere bilden können. Diese Bildung von Dimeren könnte im Falle einer Immunisierung mit NXY ausserdem dazuführen, dass erfindungsgemässe Polypeptide, die den N-terminalen Teil der Aminosäuresequenz (I) enthalten, direkt an das natürliche Plasmodium Sporozoitenantigen der vorliegenden Erfindung binden und durch diese Reaktion die Hepatozyten-Invasion erschwert oder gar hemmt.

Analyse des NXY Gens aus dem Malariaparasiten P. berghei

Mäusen wurden intravenös mit P. berghei (Isolat Anka) infiziert. Bei einer Parasitämie von 40% wurde den Mäusen das Blut entnommen. Die Isolation der Parasiten aus dem Blut erfolgte gemäss der Vorschrift von Trager et al., Science 193, 673-675 [1976].

Wie oben für das K1-Isolat von P.falciparum beschrieben wurde eine Genbank des Plasmodium berghei Genoms hergestellt. Diese P.berghei Lambda Genbank wurde nun wie oben beschrieben plattiert (2 x $10^5$ Phagenpartikel auf zwei Petrischalen von 135 mm Durchmesser). Nach fünf Stunden, als Plaques sichtbar wurden, wurden die Petrischalen aus dem 37°C Inkubator genommen und über Nacht im Kühlschrank aufbewahrt. Auf die kalten Schalen werden nun PALL-Nylonfilter (PALL, Basel, Schweiz) gelegt und die relative Position der Filter auf den Petrischalen wurde mit einem Tintenstift markiert. Nach 5 Minuten wurden die Filter vorsichtig von der Platte abgehoben und mit der Seite mit den Plaques nach oben auf Whatmann 3MM Papier gelegt, das zuvor mit alkalischer Lösung (0.5 N Natriumhydroxid und 0.5 M Tris) getränkt worden war. Nach einigen Minuten wurden die Filter auf ein neues, mit der alkalischen Lösung getränktes Whatmann 3MM Papier gelegt. Danach wurden die Filter kurz auf einem 3MM Filterpapier getrocknet und dann zweimal während fünf Minuten auf Whatman 3MM Papier gelegt, das zuvor mit 1.5 M NaCl, 0.5 M Tris/HCl [pH 8.0] getränkt worden war. Die Filter wurden dann an der Luft getrocknet und während 90 Minuten bei 80°C im Vakuum gebacken. Eine P. falciparum Probe wurde hergestellt, indem das 2 kb EcoRI-Fragment aus Klon NXY-H isoliert (Methoden 1, 2 und 3) und radioaktiv markiert wurde (Methode 11). Mit dieser P. falciparum Probe wurde ein positiver Klon (PB-B) erhalten, der isoliert (Methode 4 und 12) und analysiert wurde. Der Klon PB-B wurde nach Methode 5 vermehrt, die DNA isoliert und mit EcoRI verdaut (Methode 2). Ein 400 bp Fragment, das nicht in der Kontroll-DNA (Lambda gt11) vorhanden war, wurde isoliert, in M13 mp18 kloniert und sequenziert (Methoden 6, 7, 8 und 9). Die ersten 47 Aminosäuren nach dem ATG-Startcodon, die von der PB-B DNA kodiert werden, sind identisch mit den entsprechenden Aminosäuren des P. falciparum Sporozoitenantigens mit der N-terminalen Aminosäuresequenz (I). Diese Sequenzhomologie ist aussergewöhnlich. So gibt es zum Beispiel bei den repetitiven Sequenzen des Circumsporozoiten-Antigens CS in den verschiedenen Plasmodium-Spezies wenig Sequenzhomologie.

<u>Herstellung eines erfindungsgemässen Polypeptides in E.coli</u>

5 µg NXY DNA wurden mit 10 Einheiten EcoRI während 2 Minuten bei 37°C partiell gespalten. Das resultierende 3 kb Fragment wurde nach Methode 2 und 3 isoliert und in die EcoRI Schnittstelle von M13 mp18 eingeführt (Methode 6). Der Subklon wurde mit M13-NXY bezeichnet.

Ein für P.falciparum spezifisches Asel Fragment wurde aus dem Klon M13-NXY gemäss den Methoden 1 bis 3 wie folgt isoliert. 6 µg M13-NXY DNA wurden mit 30 Einheiten Asel in 100 µl 1x T4-Polymerasepuffer während einer Stunde bei 37°C verdaut. Die DNA wurde gefällt (Methode 1), auf einem 0,8% (w/v) Agarose Gel aufgetrennt (Methode 2) und ein 1400 bp Fragment isoliert (Methode 3). Die Enden wurden wie oben beschrieben mit Klenow Polymerase aufgefüllt. Das Fragment wurde in 20 µl Wasser resuspendiert und nach Zugabe von 10 pMolen eines phosphorylierten BamHI Oligonukleotidadaptors (10-mer: CCGGATCCGG; New England Biolabs), 2.5 µl 10 x Ligase Puffer und 6 Weiss-Einheiten T4 DNA-Ligase über Nacht bei 14°C ligiert. Die DNA wurde gefällt (Methode 1), in 50 µl 1 x T4-Polymerase Puffer gelöst und nach Zugabe von 40 Einheiten BamHI während 1 Stunde bei 37°C verdaut. Die DNA wurde gefällt (Methode 1) und auf einem 1,0% (w/v) Agarose Gel aufgetrennt (Methode 2). Ein 1400 bp Fragment wurde isoliert (Methode 3) und in 10 µl Wasser gelöst. Zur Vorbereitung des Vektors (siehe Methode 6) wurde 1 µg pDS56/RBSII,6xHis Vektor DNA mit 10 Einheiten BamHI während einer Stunde in T4-Polymerase Puffer bei 37°C verdaut. Der Vektor pDS56/RBSII,6xHis ist ein Derivat des Vektors pDS56/RBSII, der in der Europäischen Patentanmeldung, Publikations-Nr. 282 042 ausführlich beschrieben ist. Die Nukleotidsequenz des Vektors pDS56/RBSII,6xHis ist in Fig. 10 gezeigt. Der Vektor pDS56/RBSII,6xHis unterscheidet sich vom Vektor pDS56/RBSII durch ein zusätzliches DNA-Fragment, das für 6 Histidinreste kodiert. Der Vektor pDS56/RBSII,6xHis kann mittels üblicher Methoden der DNA-Rekombinationstechnik aus dem Vektor pDS56/RBSII hergestellt werden, z.B. durch Einbau eines passenden synthetischen DNA-Fragmentes in den Vektor pDS56/RBSII. Der Vektor pDS56/RBSII,6xHis ist in Form einer Kultur von E. coli M15 enthaltend die Plasmide pDS56/RBSII,6xHis sowie pDMI,1 seit dem 6. April 1989 bei der Deutschen Sammlung von Mikroorganismen, Mascheroder Weg 16 in Braunschweig, Deutschland unter der DSM-Nr. 5298, und zwar im Zusammenhang mit der Europäischen Patentanmeldung, Publikation Nr. 393 502, gemäss dem Budapester Vertrag hinter legt. Die Vektor DNA wurde dephosphoryliert (Methode 4), einmal mit Phenol extrahiert (s. oben), auf einem 0,8 % (w/v) Agarosegel gereinigt (Methode 2) und anschliessend gemäss Methode 3 isoliert. Die isolierte DNA wurde in 50 µl Wasser gelöst.

5 µl der linearisierten pDS56/RBSII,6xHis Vektor DNA, die mit BamHI verdaut und dephosphoryliert worden war (Methode 6), wurden mit 5 µl des 1400 bp Fragments, 1,2 µl 10 x Ligase Puffer und 6 Weiss Einheiten T4-DNA Ligase während einer Stunde bei Raumtemperatur inkubiert. 10 µl DNA wurden dann in kompetente E. coli SG13009 (pUHA1) Zellen transformiert (Methode 7) und auf LB-Platten mit 100 µg/ml Ampicillin und 25 µg/ml Kanamycin plattiert. Einzelne Kolonien wurden mit einem Zahnstocher gepickt und in 3 ml LB-Medium enthaltend 100 µg/ml Ampicillin und 25 µg/ml Kanamycin überführt. Die Kulturen wurden bei 37° C im Schüttelbad inkubiert bis die optische Dichte bei 600 nm ($OD_{600}$) gegenüber reinem Medium 0,6 betrug. Ein Aliquot von 500 µl der Kultur wurde als nicht-induzierte Kontrolle entnommen. Zum Rest der Kultur wurde IPTG (1 mM Endkonzentration) gegeben und die induzierte Kultur weitere 3 Stunden inkubiert. Danach wurden 500 µl der induzierten Kultur entnommen und zusammen mit der nicht-induzierten Probe zentrifugiert (3 Minuten bei 12'000 RPM, 20° C). Der Ueberstand wurde abgesaugt und das Zellsediment in 100 µl SDS-Probenpuffer resuspendiert. Die Proben wurden während 7 Minuten in kochendem Wasser inkubiert und anschliessend die Proteine auf einem 12% SDS-Polyacrylamidgel (Methode 13) mittels Elektrophorese (drei Stunden bei 50 mA konstantem Strom) aufgetrennt. Das Gel wurde während 30 Minuten mit 0,1% (w/v) Coomassie Blau in 30% (v/v) Essigsäure und 10% (v/v) Methanol auf dem Schüttler gefärbt. Entfärbt wurde das Gel während 2 Stunden bei 65° C in 10% (v/v) Methanol und 10% (v/v) Essigsäure. Klone, die gegenüber der uninduzierten Probe zusätzliche Banden mit dem scheinbaren Molekulargewicht von 69 kDa (= 69'000 Dalton) zeigten, wurden mit E. coli SG13009 (pDS-NXY; pUHA1) bezeichnet. Der Stamm E. coli SG13009 (pDS-NXY; pUHA1) wurde am 16. März 1990 bei der Deutschen Sammlung von Mikroorganismen in Braunschweig, Deutschland unter der DSM-Nr. 5846 gemäss dem Budapester Vertrag hinterlegt. Der Stamm E. coli SG13009 ist von Gottesman et al. in J. Bacteriol. 148, 265-273 (1981) beschrieben. Das Plasmid pUHA1 kodiert für den lac-Repressor. Es ist ein Derivat des Plasmids pDMI,1, das ausführlich in der Europäischen Patentanmeldung, Publikations-Nr. 309 746 beschrieben ist. Das Plasmid pUHA1 unterscheidet sich vom Plasmid pDMI,1 durch den Austausch des $lacI^q$-Allels durch das lacI-Allel das den Wildtyppromoter enthält. Durch diesen Austausch wird gewährleistet, dass im Stamm SG13009 (pDS-NXY; pUHA1) eine optimale Menge lac-Repressor hergestellt wird. Dies ermöglicht eine besonders effiziente Expression eines rekombinanten Proteins. Der Stamm SG13009 (pUHA1) kann in bekannter Art und Weise ausgehend vom Stamm SG13009 (pDS-NXY; pUHA1) erhalten werden.

Westernblot-Analyse der Expressionsprodukte von pDS-NXY

Ein Lysat von E. coli SG13009 (pDS-NXY; pUHA1) wurde auf einen Mini-SDS Gel aufgetrennt und die Proteine auf Nitrocellulosefilter übertragen (Methoden 13 und 14). Anschliessend wurde der Filter gemäss der Westernblot-Technik (Towbin et al., Proc. Natl. Acad. Sci. USA 76, 4350-4354 [1979]) unter Verwendung von Anti-Sporozoiten-Kaninchenserum oder Humanseren von Malaria exponierten Individuen analysiert. Dabei konnte gezeigt werden, dass diese Seren im Lysat Malariaparasiten-spezifische Antigene erkennen. Dies bedeutet, dass das synthetische Antigen mit der Aminosäuresequenz (II) von Antikörpern gegen das natürliche Antigen erkannt wird, d.h. dass es in mindestens einem spezifischen Epitop mit dem Plasmodium falciparum Sporozoitenantigen mit der N-terminalen Aminosäuresequenz (I) übereinstimmt. Indirekt kann auch geschlossen werden, dass das natürliche Antigen in vivo vom Immunsystem von infizierten Individuen erkannt werden kann.

Reaktion von Antikörpern gegen das rekombinante 69 kDa Protein mit Sporozoiten und Blutstadien von P.falciparum

E. coli SG13009 (pDS-NXY; pUHA1) wurde gemäss üblicher Methoden kultiviert und zur Expression des rekombinanten Polypeptides mit der Aminosäuresequenz (II) (= 69 kDa Protein) induziert. Das rekombinante 69 kDa-Protein wurde anschliessend aus dem E. coli Lysat isoliert und mittels Affinitätschromatographie an einem Nitrilotriessigsäure-Nickelchelatharz gereinigt. Das gereinigte 69 kDa Protein wurde zur Immunisation von Kaninchen benutzt. Nach zwei Booster Injektionen wurde das Serum mittels Immunofluoreszenz auf unfixierten Plasmodium Sporozoiten und Merozoiten getestet. Das Serum erkannte beide Stadien des Parasiten. Daher wurde das Kaninchenserum (Verdünnung 1:500) auf Western-Blots von Sporozoiten- und Blutstadienextrakten getestet. Im Sporozoitenextrakt erkannte das Serum eine Bande von über 200 kDa, während im Blutstadienextrakt drei Banden von etwa 50-55 kDa erkannt wurden. 50-53 kDa Proteine aus Blutstadien sind von Wahlgren et al., Proc. Natl. Acad. Sci. USA 83, 2677-2681 [1986] beschrieben. Ein Vergleich der Aminosäuresequenz dieser 50-53 kDa Proteine mit der Aminosäuresequenz des Sporozoitenantigens der vorliegenden Erfindung zeigt, dass es sich um unterschiedliche Proteine handeln muss. Ausserdem wurde gefunden, dass unter Verwendung der NXY-DNA als Probe in Blutstadien RNA keine NXY-Transkripte nachweisbar sind. Damit ist das erfindungsgemässe Plasmodium Antigen spezifisch für das Sporozoitenstadium.

Erkennung des NXY Gens in 9 P.falciparum Isolaten und in P.berghei

Genomische DNA von 9 verschiedenen P.falciparum Isolaten wurde jeweils mit SspI verdaut und die Fragmente auf einem 1,2% (w/v) Agarose Gel getrennt. Die DNAs wurden auf Nitrocellulose Membran übertragen und mit einer radioaktiven Plasmodium Sporozoitenantigen-Gen-spezifischen Probe aus der M13-NXY-DNA (2 kb EcoRI-Fragment) hybridisiert (60° C, 2xSSC; Methoden 2, 3, 12, 15). Fig. 9 Teil A zeigt klar, dass bei allen 9 Isolaten, die analysiert wurden, zwei Banden (ca. 1,7 kb und ca. 15 kb relativ zu den Markern) nachgewiesen werden können. Unterschiede in der Intensität sind durch unterschiedliche Mengen DNA bedingt.

Um zu testen ob das NXY-Gen auch im Mausmalariaparasit P.berghei nachgewiesen werden kann, wurden jeweils P.falciparum DNA (Spuren 1 und 2) und P.berghei DNA (Spuren 3 und 4) mit DraI (Spuren 1 und 3) oder HindIII (Spuren 2 und 4) geschnitten und wie oben mit der radioaktiven Probe aus der M13-NXY-DNA getestet. Figur 9 Teil B zeigt klar, dass in allen 4 Spuren eine NXY-spezifische Bande nachgewiesen werden kann. Obwohl die nachgewiesene NXY-spezifische DNA jeweils unterschiedliche Grösse aufweist, handelt es sich aber nicht um eine unspezifische Kreuzreaktion, da, wie oben gezeigt wurde, die für die ersten 47 Aminosäuren kodierende Nukleotidsequenz in der P. falciparum DNA gleich ist wie die entsprechende Nukleotidsequenz in der P. berghei DNA.

**Patentansprüche**

1. Polypeptide die in mindestens einem spezifischen Epitop mit dem Plasmodium falciparum Sporozoitenantigen, das die N-terminale Aminosäuresequenz (I)

```
                   5                       10                      15
                   |                        |                       |
  1 Met Asn Lys Val Asn Ala Val His Lys Ile Asn Ala Val Asp Lys
 16 Val Asn Ala Val Asn Lys Val Asn Ser Val Asn Lys Leu Asn Val
 31 Val Asn Lys Thr Asn Val Leu Ser Lys Leu Asn Ala Val Tyr Lys
 46 Val Asn Ser Val His Lys Met Asn Ala Val Asn Lys Val Asn Ala
 61 Val Asn Lys Val Asn Ala Val Asn Lys Val Asn Val Val Asn Lys
 76 Lys Asp Ile Leu Asn Lys Leu Asn Ala Leu Tyr Lys Met Asn Ala
 91 Val Tyr Lys Met Asn Ala Leu Asn Lys Val Ser Ala Val Asn Lys
106 Val Ser Ala Val Asn Lys Val Ser Ala Val Asn Lys Met Gly Ala
121 Val Asn Arg Val Asn Gly Val Asn Lys Val Asn Glu Val Asn Glu
136 Val Asn Glu Val Asn Glu Val Asn Met Val Asn Glu Val Asn Glu
151 Leu Asn Glu Val Asn Asn Val Asn Ala Val Asn Glu Val Asn Ser
166 Val Asn Glu Val Asn Glu Met Asn Glu Val Asn Lys Val Asn Glu
181 Leu Asn Glu Val Asn Glu Val Asn Asn Val Asn Glu Val Asn Asn
196 Val Asn Val Met Asn Asn Val Asn Glu Met Asn Asn Met Asn Glu
211 Met Asn Asn Val Asn Val Val Asn Glu Val Asn Asn Val Asn Glu
226 Val Asn Asn Val Asn Glu Met Asn Asn Val Asn Glu Met Asn Asn
241 Met Asn Glu Met Asn Asn Val Asn Val Val Asn Glu Val Asn Asn
256 Val Asn Glu Met Asn Asn Thr Asn Glu Leu Asn Glu Val Asn Glu
271 Val Asn Asn Val Asn Glu Val Asn Asp Val Asn Val Val Asn Glu
286 Val Asn Asn Val Asn Glu Met Asn Asn Met Asn Glu Leu Asn Glu
301 Val Asn Gly Val Asn Glu Val Asn Asn Thr Asn Glu Ile His Glu
316 Met Asn Asn Ile Asn Glu Val Asn Asn Thr Asn Glu Val Asn Asn
331 Thr Asn Glu Ile Tyr Glu Met Asn Asn Met Asn Asp Val Asn Asn
346 Thr Asn Glu Ile Asn Val Val Asn Ala Val Asn Glu Val Asn Lys
361 Val Asn Asp Ser Asn Asn Ser Asn Asp Ala Asn Glu Gly Asn Asn
376 Ala Asn Tyr Ser Asn Asp Ser Ser Asn Thr Asn Asn Asn Thr Ser
391 Ser Ser Thr Asn Asn Ser Asn Asn Asn Thr Ser Cys Ser Ser Gln
406 Asn Thr Thr Thr Ser Ser Glu Asn Asn Asp Ser Leu Glu Asn Lys
421 Arg Asn Glu Glu Asp Glu Asp Glu Glu Asp Asp Gln Lys Asp Thr
436 Gln Lys Glu Lys Asn Asn Leu Glu Gln Glu Asp Met Ser Pro Tyr
451 Glu Asp Arg Asn Lys Asn Asp Glu Lys Asn Ile Asn Glu Gln Asp
466 Lys Phe His Leu Ser Asn Asp Leu Gly Lys Ile Tyr Asp Thr Tyr
481 Asn Gln Gly Asp Glu Val Val Val Ser Lys Asn Lys Asp Lys Leu
496 Glu Lys His Leu Asn Asp Tyr Lys Ser Tyr Tyr Tyr Leu Ser Lys
511 Ala Thr Leu Met Asp Lys Ile Gly Glu Ser Gln Asn Asn Asn Asn
526 Tyr Asn Val Cys Asn Ser Asn Glu Leu Gly Thr Asn Glu Ser Ile
541 Lys Thr Asn Ser Asp Gln Asn Asp Asn Val Lys Glu Lys Asn Asp
556 Ser Asn Ile Phe Met Lys Met Ile Ile Ile Ile Arg Leu Met Ile
571 Met Ile Ile Met Ile Met Ile Ile Ile Trp Tyr Leu Lys Ile Leu
586 Gln Asp Lys Ile Ile Trp Arg Asn Lys Lys Val Glu Lys Thr Ser
601 Asn Ile Leu Asn Asn Phe Asp Asn Asn Gly Asn Asp Asn Asp Asn
616 Asp Asn Asp Asp Asn Asn Asp Asn Asp Asn Asn Asn Asn Asn Asn
631 Met Asn Asn Gln Tyr Asn Tyr Gln Glu Asn Asn Ile Asn Thr Asn
646 Tyr Asn Ile Leu Tyr Thr Pro Ser Asn Cys Gln Ile Gln Asn Asn
661 Ser Tyr Met Asn Thr Asn Glu Met Tyr Gln Pro Leu Tyr Asn Thr
676 Tyr Pro Ser Asn Arg Ile Gln Glu Asn Ser Thr Ile Asn Asn Asn
691 Ile Ile Asn Asp Ser Pro Tyr Met Asn Asn Asp Asn Thr Thr Asn
706 Asn Thr Phe Ile Ser Gly Met Asn
```

enthält, übereinstimmen.

2. Polypeptide gemäss Anspruch 1 die kovalent mit einem Affinitätspeptid verknüpft sind, z.B. das Polypeptid mit der Aminosäuresequenz (II)

```
                          5                   10                  15
                          |                   |                   |
  1 Met Arg Gly Ser His His His His His His Gly Ser Val Asn Ser
 16 Val Asn Lys Glu Asn Asn Met Asn Lys Val Asn Ala Val His Lys
 31 Ile Asn Ala Val Asp Lys Val Asn Ala Val Asn Lys Val Asn Ser
 46 Val Asn Lys Leu Asn Val Val Asn Lys Thr Asn Val Leu Ser Lys
 61 Leu Asn Ala Val Tyr Lys Val Asn Ser Val His Lys Met Asn Ala
 76 Val Asn Lys Val Asn Ala Val Asn Lys Val Asn Ala Val Asn Lys
 91 Val Asn Val Val Asn Lys Lys Asp Ile Leu Asn Lys Leu Asn Ala
106 Leu Tyr Lys Met Asn Ala Val Tyr Lys Met Asn Ala Leu Asn Lys
121 Val Ser Ala Val Asn Lys Val Ser Ala Val Asn Lys Val Ser Ala
136 Val Asn Lys Met Gly Ala Val Asn Arg Val Asn Gly Val Asn Lys
151 Val Asn Glu Val Asn Glu Val Asn Glu Val Asn Glu Val Asn Met
166 Val Asn Glu Val Asn Glu Leu Asn Glu Val Asn Asn Val Asn Ala
181 Val Asn Glu Val Asn Ser Val Asn Glu Val Asn Glu Met Asn Glu
196 Val Asn Lys Val Asn Glu Leu Asn Glu Val Asn Glu Val Asn Asn.
211 Val Asn Glu Val Asn Asn Val Asn Val Met Asn Asn Val Asn Glu
226 Met Asn Asn Met Asn Glu Met Asn Asn Val Asn Val Val Asn Glu
241 Val Asn Asn Val Asn Glu Val Asn Asn Val Asn Glu Met Asn Asn
256 Val Asn Glu Met Asn Asn Met Asn Glu Met Asn Asn Val Asn Val
271 Val Asn Glu Val Asn Asn Val Asn Glu Met Asn Asn Thr Asn Glu
286 Leu Asn Glu Val Asn Glu Val Asn Asn Val Asn Glu Val Asn Asp
301 Val Asn Val Val Asn Glu Val Asn Asn Val Asn Glu Met Asn Asn
316 Met Asn Glu Leu Asn Glu Val Asn Gly Val Asn Glu Val Asn Asn
331 Thr Asn Glu Ile His Glu Met Asn Asn Ile Asn Glu Val Asn Asn
346 Thr Asn Glu Val Asn Asn Thr Asn Glu Ile Tyr Glu Met Asn Asn
361 Met Asn Asp Val Asn Asn Thr Asn Glu Ile Asn Val Val Asn Ala
376 Val Asn Glu Val Asn Lys Val Asn Asp Ser Asn Asn Ser Asn Asp
391 Ala Asn Glu Gly Asn Asn Ala Asn Tyr Ser Asn Asp Ser Ser Asn
406 Thr Asn Asn Asn Thr Ser Ser Ser Thr Asn Asn Ser Asn Asn Asn
421 Thr Ser Cys Ser Ser Gln Asn Thr Thr Thr Ser Ser Glu Asn Asn
436 Asp Ser Leu Glu Asn Lys Arg Asn Glu Glu Asp Glu Asp Glu Glu
451 Asp Asp Gln Lys Asp Thr Gln Lys Glu Lys Asn Asn Leu Glu Gln
466 Glu Asp Met Ser Pro Tyr Glu Asp Arg Asn Lys Asn Asp Glu Lys
481 Asn Ile Asn Gly Ile Arg Arg Pro Ala Ala Lys Leu Asn
```

**3.** Polypeptide gemäss Anspruch 1 oder 2 mit einer Teilsequenz

$(NXY)_n$, $(YNX)_n$ oder $(XYN)_n$

worin

- N ein Asparaginrest,
- X ein geladener Aminosäurerest, z.B. ein Aminosäurerest von Glutaminsäure oder Lysin,
- Y ein hydrophober Aminosäurerest, z.B. ein Aminosäurerest von Valin oder Methionin und
- n eine Zahl zwischen 3 und 120 ist.

**4.** Polypeptide gemäss Anspruch 1, 2 oder 3, die an ein Trägermaterial adsorbiert oder kovalent daran gebunden sind.

**5.** Eine DNA, die für ein Polypeptid gemäss Anspruch 1, 2 oder 3 kodiert, insbesondere eine DNA, die die Nukleotidsequenz von Position 948 bis Position 3088 in Figur 2 oder eine Teilsequenz davon enthält.

**6.** Ein replizierbarer mikrobieller Vektor, der eine DNA enthält, die für ein Polypeptid gemäss Anspruch 1, 2 oder 3 kodiert, insbesondere eine DNA, die die Nukleotidsequenz von Position 948 bis Position 3088 in Figur 2 oder eine Teilsequenz davon enthält, wobei die DNA bevorzugt so an eine Expressionskontrollsequenz gebunden ist, dass das von der DNA kodierte Polypeptid exprimiert werden kann.

7. Ein transformierter Mikroorganismus, der einen replizierbaren mikrobiellen Vektor enthält, wobei der Vektor eine DNA enthält, die für ein Polypeptid gemäss Anspruch 1, 2 oder 3 kodiert, insbesondere eine DNA, die die Nukleotidsequenz von Position 948 bis Position 3088 in Figur 2 oder eine Teilsequenz davon enthält, wobei die DNA bevorzugt so an eine Expressionskontrollsequenz gebunden ist, dass das von der DNA kodierte Polypeptid exprimiert werden kann.

8. Antikörper die gegen ein Polypeptid gemäss Anspruch 1, 2 oder 3 gerichtet sind.

9. Polypeptide gemäss einem der Ansprüche 1 bis 4 für die Immunisierung von Säugern gegen Malaria.

10. Ein Verfahren zur Herstellung von Polypeptiden gemäss Anspruch 1, 2 oder 3, dadurch gekennzeichnet, dass:
(a) ein Mikroorganismus, der mit einem replizierbaren mikrobiellen Vektor, der eine DNA enthält, die für ein Polypeptid gemäss Anspruch 1, 2 oder 3 kodiert, insbesondere eine DNA, die die Nukleotidsequenz von Position 948 bis Position 3088 in Figur 2 oder eine Teilsequenz davon enthält, transformiert worden ist, unter Bedingungen kultiviert wird, die die Expression des von der DNA kodierten Polypeptids erlauben; und
(b) das Polypeptid aus der Kultur isoliert wird.

11. Ein Verfahren zur Herstellung von Polypeptiden gemäss Anspruch 1, 2 oder 3, dadurch gekennzeichnet, dass:
(a) Peptide mittels konventioneller Methoden der Peptidsynthese hergestellt werden; und
(b) mehrere Peptide in der gewünschten Reihenfolge miteinander verknüpft werden.

12. Ein Verfahren zur Herstellung eines replizierbaren mikrobiellen Vektors, der eine DNA enthält, die für ein Polypeptid gemäss Anspruch 1, 2 oder 3 kodiert, insbesondere eine DNA, die die Nukleotidsequenz von Position 948 bis Position 3088 in Figur 2 oder eine Teilsequenz davon enthält, dadurch gekennzeichnet, dass:
(a) eine DNA, die für ein Polypeptid gemäss Anspruch 1, 2 oder 3 kodiert, insbesondere eine DNA, die die Nukleotidsequenz von Position 948 bis Position 3088 in Figur 2 oder eine Teilsequenz davon enthält, in einen Vektor integriert wird;
(b) der transformierte Vektor in einem Mikroorganismus repliziert wird; und
(c) die replizierten mikrobiellen Vektoren aus dem Mikroorganismus isoliert werden.

13. Ein Verfahren zur Herstellung eines transformierten Mikroorganismus der fähig ist ein Polypeptid gemäss Anspruch 1, 2 oder 3 zu produzieren, dadurch gekennzeichnet, dass:
(a) ein Mikroorganismus, der einen replizierbaren mikrobiellen Vektor enthält, wobei der Vektor eine DNA enthält, die für ein Polypeptid gemäss Anspruch 1, 2 oder 3 kodiert, insbesondere eine DNA, die die Nukleotidsequenz von Position 948 bis Position 3088 in Figur 2 oder eine Teilsequenz davon enthält, wobei die DNA so an eine Expressionskontrollsequenz gebunden ist, dass das von der DNA kodierte Polypeptid exprimiert werden kann, transformiert wird; und
(b) der transformierte Mikroorganismus in einem Kulturmedium vermehrt wird.

14. Ein Verfahren zur Herstellung von Antikörpern, die gegen ein Polypeptid gemäss Anspruch 1, 2 oder 3 gerichtet sind, dadurch gekennzeichnet, dass
(a) ein Polypeptid gemäss einem der Ansprüche 1, 2, 3 oder 4, in einen geeigneten Wirtsorganismus, der zu einer Immunreaktion gegen das Polypeptid fähig ist, injiziert wird; und
(b) die sich bildenden, gegen das Polypeptid gerichteten Antikörper auf bekannte Art und Weise isoliert werden.

15. Immunogene Kompositionen die ein Polypeptid gemäss einem der Ansprüche 1 bis 4 und ein geeignetes Adjuvans enthalten.

16. Immunogene Kompositionen gemäss Anspruch 15, als ein Vakzin.

17. Verwendung eines Polypeptids gemäss einem der Ansprüche 1 bis 4 oder einer immunogenen Komposition gemäss einem der Ansprüche 15 oder 16 zur Immunisierung von Säugern gegen Malaria.

18. Eine Methode zur Immunisierung von Säugern gegen Malaria, dadurch gekennzeichnet, dass das Immunsystem dieser Säuger mit einer immunisierenden Menge eines Polypeptids gemäss einem der Ansprüche 1 bis 4 oder einer immunogenen Zusammensetzung gemäss einem der Ansprüche 15 oder 16 stimuliert wird.

**Patentansprüche für folgenden Vertragsstaat: ES**

1. Ein Verfahren zur Herstellung eines Polypeptids, das in mindestens einem spezifischen Epitop mit dem Plasmodium falciparum Sporozoitenantigen, das die N-terminale Aminosäuresequenz (I)

```
                        5                   10                  15
                        |                   |                   |
  1 Met Asn Lys Val Asn Ala Val His Lys Ile Asn Ala Val Asp Lys
 16 Val Asn Ala Val Asn Lys Val Asn Ser Val Asn Lys Leu Asn Val
 31 Val Asn Lys Thr Asn Val Leu Ser Lys Leu Asn Ala Val Tyr Lys
 46 Val Asn Ser Val His Lys Met Asn Ala Val Asn Lys Val Asn Ala
 61 Val Asn Lys Val Asn Ala Val Asn Lys Val Asn Val Val Asn Lys
 76 Lys Asp Ile Leu Asn Lys Leu Asn Ala Leu Tyr Lys Met Asn Ala
 91 Val Tyr Lys Met Asn Ala Leu Asn Lys Val Ser Ala Val Asn Lys
106 Val Ser Ala Val Asn Lys Val Ser Ala Val Asn Lys Met Gly Ala
121 Val Asn Arg Val Asn Gly Val Asn Lys Val Asn Glu Val Asn Glu
136 Val Asn Glu Val Asn Glu Val Asn Met Val Asn Glu Val Asn Glu
151 Leu Asn Glu Val Asn Asn Val Asn Ala Val Asn Glu Val Asn Ser
166 Val Asn Glu Val Asn Glu Met Asn Glu Val Asn Lys Val Asn Glu
181 Leu Asn Glu Val Asn Glu Val Asn Asn Val Asn Glu Val Asn Asn
196 Val Asn Val Met Asn Asn Val Asn Glu Met Asn Asn Met Asn Glu
211 Met Asn Asn Val Asn Val Val Asn Glu Val Asn Asn Val Asn Glu
226 Val Asn Asn Val Asn Glu Met Asn Asn Val Asn Glu Met Asn Asn
241 Met Asn Glu Met Asn Asn Val Asn Val Val Asn Glu Val Asn Asn
256 Val Asn Glu Met Asn Asn Thr Asn Glu Leu Asn Glu Val Asn Glu
271 Val Asn Asn Val Asn Glu Val Asn Asp Val Asn Val Val Asn Glu
286 Val Asn Asn Val Asn Glu Met Asn Asn Met Asn Glu Leu Asn Glu
301 Val Asn Gly Val Asn Glu Val Asn Asn Thr Asn Glu Ile His Glu
316 Met Asn Asn Ile Asn Glu Val Asn Asn Thr Asn Glu Val Asn Asn
331 Thr Asn Glu Ile Tyr Glu Met Asn Asn Met Asn Asp Val Asn Asn
346 Thr Asn Glu Ile Asn Val Val Asn Ala Val Asn Glu Val Asn Lys
361 Val Asn Asp Ser Asn Asn Ser Asn Asp Ala Asn Glu Gly Asn Asn
376 Ala Asn Tyr Ser Asn Asp Ser Ser Asn Thr Asn Asn Asn Thr Ser
391 Ser Ser Thr Asn Asn Ser Asn Asn Asn Thr Ser Cys Ser Ser Gln
406 Asn Thr Thr Thr Ser Ser Glu Asn Asn Asp Ser Leu Glu Asn Lys
421 Arg Asn Glu Glu Asp Glu Asp Glu Glu Asp Asp Gln Lys Asp Thr
436 Gln Lys Glu Lys Asn Asn Leu Glu Gln Glu Asp Met Ser Pro Tyr
451 Glu Asp Arg Asn Lys Asn Asp Glu Lys Asn Ile Asn Glu Gln Asp
466 Lys Phe His Leu Ser Asn Asp Leu Gly Lys Ile Tyr Asp Thr Tyr
481 Asn Gln Gly Asp Glu Val Val Val Ser Lys Asn Lys Asp Lys Leu
496 Glu Lys His Leu Asn Asp Tyr Lys Ser Tyr Tyr Tyr Leu Ser Lys
511 Ala Thr Leu Met Asp Lys Ile Gly Glu Ser Gln Asn Asn Asn Asn
526 Tyr Asn Val Cys Asn Ser Asn Glu Leu Gly Thr Asn Glu Ser Ile
541 Lys Thr Asn Ser Asp Gln Asn Asp Asn Val Lys Glu Lys Asn Asp
556 Ser Asn Ile Phe Met Lys Met Ile Ile Ile Ile Arg Leu Met Ile
571 Met Ile Ile Met Ile Met Ile Ile Ile Trp Tyr Leu Lys Ile Leu
586 Gln Asp Lys Ile Ile Trp Arg Asn Lys Lys Val Glu Lys Thr Ser
601 Asn Ile Leu Asn Asn Phe Asp Asn Asn Gly Asn Asp Asn Asp Asn
616 Asp Asn Asp Asp Asn Asn Asp Asn Asp Asn Asn Asn Asn Asn Asn
631 Met Asn Asn Gln Tyr Asn Tyr Gln Glu Asn Asn Ile Asn Thr Asn
646 Tyr Asn Ile Leu Tyr Thr Pro Ser Asn Cys Gln Ile Gln Asn Asn
661 Ser Tyr Met Asn Thr Asn Glu Met Tyr Gln Pro Leu Tyr Asn Thr
676 Tyr Pro Ser Asn Arg Ile Gln Glu Asn Ser Thr Ile Asn Asn Asn
691 Ile Ile Asn Asp Ser Pro Tyr Met Asn Asn Asp Asn Thr Thr Asn
706 Asn Thr Phe Ile Ser Gly Met Asn
```

enthält, übereinstimmt, dadurch gekennzeichnet, dass:

(a) ein Mikroorganismus, der mit einem replizierbaren mikrobiellen Vektor, der eine DNA enthält, die für das genannte Polypeptid kodiert, insbesondere eine DNA die die Nukleotidsequenz von Position 948 bis Position 3088 in Figur 2 oder eine Teilsequenz davon enthält, wobei die DNA so an eine Expressionskontrollsequenz gebunden ist, dass das von der DNA kodierte Polypeptid exprimiert werden kann, transformiert worden ist, unter Bedingungen kultiviert wird, die die Expression des von der DNA kodierten Polypeptids erlauben; und
(b) das Polypeptid aus der Kultur isoliert wird.

**2.** Ein Verfahren zur Herstellung eines Polypeptids, das in mindestens einem spezifischen Epitop mit dem Plasmodium Sporozoitenantigen, das die N-terminale Aminosäuresequenz (I) enthält, übereinstimmt und kovalent mit einem Affinitätspeptid verknüpft ist, wie das Polypeptid mit der Aminosäuresequenz (II)

```
                        5                   10                  15
                        |                   |                   |
  1 Met Arg Gly Ser His His His His His His Gly Ser Val Asn Ser
 16 Val Asn Lys Glu Asn Asn Met Asn Lys Val Asn Ala Val His Lys
 31 Ile Asn Ala Val Asp Lys Val Asn Ala Val Asn Lys Val Asn Ser
 46 Val Asn Lys Leu Asn Val Val Asn Lys Thr Asn Val Leu Ser Lys
 61 Leu Asn Ala Val Tyr Lys Val Asn Ser Val His Lys Met Asn Ala
 76 Val Asn Lys Val Asn Ala Val Asn Lys Val Asn Ala Val Asn Lys
 91 Val Asn Val Val Asn Lys Lys Asp Ile Leu Asn Lys Leu Asn Ala
106 Leu Tyr Lys Met Asn Ala Val Tyr Lys Met Asn Ala Leu Asn Lys
121 Val Ser Ala Val Asn Lys Val Ser Ala Val Asn Lys Val Ser Ala
136 Val Asn Lys Met Gly Ala Val Asn Arg Val Asn Gly Val Asn Lys
151 Val Asn Glu Val Asn Glu Val Asn Glu Val Asn Glu Val Asn Met
166 Val Asn Glu Val Asn Glu Leu Asn Glu Val Asn Asn Val Asn Ala
181 Val Asn Glu Val Asn Ser Val Asn Glu Val Asn Glu Met Asn Glu
196 Val Asn Lys Val Asn Glu Leu Asn Glu Val Asn Glu Val Asn Asn
211 Val Asn Glu Val Asn Asn Val Asn Val Met Asn Asn Val Asn Glu
226 Met Asn Asn Met Asn Glu Met Asn Asn Val Asn Val Val Asn Glu
241 Val Asn Asn Val Asn Glu Val Asn Asn Val Asn Glu Met Asn Asn
256 Val Asn Glu Met Asn Asn Met Asn Glu Met Asn Asn Val Asn Val
271 Val Asn Glu Val Asn Asn Val Asn Glu Met Asn Asn Thr Asn Glu
286 Leu Asn Glu Val Asn Glu Val Asn Asn Val Asn Glu Val Asn Asp
301 Val Asn Val Val Asn Glu Val Asn Asn Val Asn Glu Met Asn Asn
316 Met Asn Glu Leu Asn Glu Val Asn Gly Val Asn Glu Val Asn Asn
331 Thr Asn Glu Ile His Glu Met Asn Asn Ile Asn Glu Val Asn Asn
346 Thr Asn Glu Val Asn Asn Thr Asn Glu Ile Tyr Glu Met Asn Asn
361 Met Asn Asp Val Asn Asn Thr Asn Glu Ile Asn Val Val Asn Ala
376 Val Asn Glu Val Asn Lys Val Asn Asp Ser Asn Asn Ser Asn Asp
391 Ala Asn Glu Gly Asn Asn Ala Asn Tyr Ser Asn Asp Ser Ser Asn
406 Thr Asn Asn Asn Thr Ser Ser Ser Thr Asn Asn Ser Asn Asn Asn
421 Thr Ser Cys Ser Ser Gln Asn Thr Thr Thr Ser Ser Glu Asn Asn
436 Asp Ser Leu Glu Asn Lys Arg Asn Glu Glu Asp Glu Asp Glu Glu
451 Asp Asp Gln Lys Asp Thr Gln Lys Glu Lys Asn Asn Leu Glu Gln
466 Glu Asp Met Ser Pro Tyr Glu Asp Arg Asn Lys Asn Asp Glu Lys
481 Asn Ile Asn Gly Ile Arg Arg Pro Ala Ala Lys Leu Asn
```

dadurch gekennzeichnet, dass:
(a) ein Mikroorganismus, der mit einem replizierbaren mikrobiellen Vektor, der eine DNA enthält, die für das genannte Polypeptid kodiert, insbesondere eine DNA die die Nukleotidsequenz von Position 948 bis Position 3088 in Figur 2 oder eine Teilsequenz davon enthält, so an eine Expressionskontrollsequenz gebunden ist, dass das von der DNA kodierte Polypeptid exprimiert werden kann, transformiert worden ist, unter Bedingungen kultiviert wird, die die Expression des von der DNA kodierten Polypeptids erlauben; und

(b) das Polypeptid aus der Kultur isoliert wird.

3. Ein Verfahren zur Herstellung eines Polypeptids gemäss Anspruch 1 oder 2, dadurch gekennzeichnet, dass das Polypeptid eine Teilsequenz

$(NXY)_n$, $(YNX)_n$ oder $(XYN)_n$

worin
N ein Asparaginrest,
X ein geladener Aminosäurerest, z.B. ein Aminosäurerest von Glutaminsäure oder Lysin,
Y ein hydrophober Aminosäurerest, z.B. ein Aminosäurerest von Valin oder Methionin und
n eine Zahl zwischen 3 und 120 ist,
enthält.

4. Ein Verfahren zur Herstellung eines Polypeptids wie es in einem der Ansprüche 1, 2 oder 3 definiert ist, dadurch gekennzeichnet, dass
(a) Peptide mittels konventioneller Methoden der Peptidsynthese hergestellt werden; und
(b) mehrere Peptide in der gewünschten Reihenfolge miteinander verknüpft werden.

5. Ein Verfahren zur Herstellung eines replizierbaren mikrobiellen Vektors, der eine DNA enthält, die für ein Polypeptid wie es in Anspruch 1, 2 oder 3 definiert ist, insbesondere eine DNA, die die Nukleotidsequenz von Position 948 bis Position 3088 in Figur 2 oder eine Teilsequenz davon enthält, dadurch gekennzeichnet, dass:
(a) eine DNA, die für ein Polypeptid wie es in Anspruch 1, 2 oder 3 definiert ist, insbesondere eine DNA, die die Nukleotidsequenz von Position 948 bis Position 3088 in Figur 2 oder eine Teilsequenz davon enthält, in einen Vektor integriert wird;
(b) der transformierte Vektor in einem Mikroorganismus repliziert wird; und
(c) die replizierten mikrobiellen Vektoren aus dem Mikroorganismus isoliert werden.

6. Ein Verfahren zur Herstellung eines transformierten Mikroorganismus, der fähig ist ein Polypeptid, wie es in einem der Ansprüche 1, 2 oder 3 definiert ist, zu produzieren, dadurch gekennzeichnet, dass:
(a) ein Mikroorganismus, der einen replizierbaren mikrobiellen Vektor enthält, wobei der Vektor eine DNA enthält, die für ein Polypeptid wie es in Anspruch 1, 2 oder 3 definiert ist, insbesondere eine DNA, die die Nukleotidsequenz von Position 948 bis Position 3088 in Figur 2 oder eine Teilsequenz davon enthält, wobei die DNA so an eine Expressionskontrollsequenz gebunden ist, dass das von der DNA kodierte Polypeptid exprimiert werden kann, transformiert wird; und
(b) der transformierte Mikroorganismus in einem Kulturmedium vermehrt wird.

7. Ein Verfahren zur Herstellung von Antikörpern, die gegen ein Polypeptid, wie es in einem der Ansprüche 1, 2 oder 3 definiert ist, gerichtet sind, dadurch gekennzeichnet, dass:
(a) ein Polypeptid wie es in einem der Ansprüche 1, 2 oder 3 definiert ist, in einem geeigneten Wirtsorganismus, der zu einer Immunreaktion gegen das Polypeptid fähig ist, injiziert wird; und
(b) und die sich bildenden, gegen das Polypeptid gerichteten Antikörper auf bekannte Art und Weise isoliert werden.

8. Ein Verfahren zur Herstellung eines Anti-Malariavakzins, dadurch gekennzeichnet, dass ein gemäss einem Verfahren nach Anspruch 1, 2 oder 3 hergestelltes Polypeptid mit einem pharmazeutisch zulässigen Träger oder einem Adjuvans gemischt wird.

9. Verwendung eines Polypeptids, wie es in einem der Ansprüche 1, 2 oder 3 definiert ist, für die Herstellung eines Anti-Malariavakzins.

10. Eine DNA, die für ein Polypeptid, wie es in einem der Ansprüche 1, 2 oder 3 definiert ist, kodiert, insbesondere eine DNA, die die Nukleotidsequenz von Position 948 bis Position 3088 in Figur 2 oder eine Teilsequenz davon enthält.

11. Ein replizierbarer Vektor, der eine DNA enthält, die für ein Polypeptid, wie es in einem der Ansprüche 1, 2 oder 3 definiert ist, kodiert, insbesondere eine DNA, die die Nukleotidsequenz von Position 948 bis Position 3088 in Figur 2 oder eine Teilsequenz davon enthält, wobei die DNA bevorzugt so an eine Expressionskontrollsequenz gebunden ist, dass das von der DNA kodierte Polypeptid exprimiert werden kann.

**12.** Ein transformierter Mikroorganismus, der einen replizierbaren mikrobiellen Vektor enthält, wobei der Vektor eine DNA enthält, die für ein Polypeptid, wie es in einem der Ansprüche 1, 2 oder 3 definiert ist, kodiert, insbesondere eine DNA, die die Nukleotidsequenz von Position 948 bis Position 3088 in Figur 2 oder eine Teilsequenz davon enthält, wobei die DNA bevorzugt so an eine Expressionskontrollsequenz gebunden ist, dass das von der DNA kodierte Polypeptid exprimiert werden kann.

**13.** Antikörper die gegen ein Polypeptid, wie es in einem der Ansprüche 1, 2 oder 3 definiert ist, gerichtet sind.

**14.** Eine Methode zur Immunisierung von Säugern gegen Malaria, dadurch gekennzeichnet, dass das Immunsystem dieser Säuger mit einer immunisierenden Menge eines Polypeptids wie es in einem der Ansprüche 1, 2 oder 3 definiert ist, stimuliert wird.

# FIG 1

| | | | | | 5 | | | | | 10 | | | | | 15 |
|---|---|---|---|---|---|---|---|---|---|---|---|---|---|---|---|
| 1 | Met | Asn | Lys | Val | Asn | Ala | Val | His | Lys | Ile | Asn | Ala | Val | Asp | Lys |
| 16 | Val | Asn | Ala | Val | Asn | Lys | Val | Asn | Ser | Val | Asn | Lys | Leu | Asn | Val |
| 31 | Val | Asn | Lys | Thr | Asn | Val | Leu | Ser | Lys | Leu | Asn | Ala | Val | Tyr | Lys |
| 46 | Val | Asn | Ser | Val | His | Lys | Met | Asn | Ala | Val | Asn | Lys | Val | Asn | Ala |
| 61 | Val | Asn | Lys | Val | Asn | Ala | Val | Asn | Lys | Val | Asn | Val | Val | Asn | Lys |
| 76 | Lys | Asp | Ile | Leu | Asn | Lys | Leu | Asn | Ala | Leu | Tyr | Lys | Met | Asn | Ala |
| 91 | Val | Tyr | Lys | Met | Asn | Ala | Leu | Asn | Lys | Val | Ser | Ala | Val | Asn | Lys |
| 106 | Val | Ser | Ala | Val | Asn | Lys | Val | Ser | Ala | Val | Asn | Lys | Met | Gly | Ala |
| 121 | Val | Asn | Arg | Val | Asn | Gly | Val | Asn | Lys | Val | Asn | Glu | Val | Asn | Glu |
| 136 | Val | Asn | Glu | Val | Asn | Glu | Val | Asn | Met | Val | Asn | Glu | Val | Asn | Glu |
| 151 | Leu | Asn | Glu | Val | Asn | Asn | Val | Asn | Ala | Val | Asn | Glu | Val | Asn | Ser |
| 166 | Val | Asn | Glu | Val | Asn | Glu | Met | Asn | Glu | Val | Asn | Lys | Val | Asn | Glu |
| 181 | Leu | Asn | Glu | Val | Asn | Glu | Val | Asn | Asn | Val | Asn | Glu | Val | Asn | Asn |
| 196 | Val | Asn | Val | Met | Asn | Asn | Val | Asn | Glu | Met | Asn | Asn | Met | Asn | Glu |
| 211 | Met | Asn | Asn | Val | Asn | Val | Val | Asn | Glu | Val | Asn | Asn | Val | Asn | Glu |
| 226 | Val | Asn | Asn | Val | Asn | Glu | Met | Asn | Asn | Val | Asn | Glu | Met | Asn | Asn |
| 241 | Met | Asn | Glu | Met | Asn | Asn | Val | Asn | Val | Val | Asn | Glu | Val | Asn | Asn |
| 256 | Val | Asn | Glu | Met | Asn | Asn | Thr | Asn | Glu | Leu | Asn | Glu | Val | Asn | Glu |
| 271 | Val | Asn | Asn | Val | Asn | Glu | Val | Asn | Asp | Val | Asn | Val | Val | Asn | Glu |
| 286 | Val | Asn | Asn | Val | Asn | Glu | Met | Asn | Asn | Met | Asn | Glu | Leu | Asn | Glu |
| 301 | Val | Asn | Gly | Val | Asn | Glu | Val | Asn | Asn | Thr | Asn | Glu | Ile | His | Glu |
| 316 | Met | Asn | Asn | Ile | Asn | Glu | Val | Asn | Asn | Thr | Asn | Glu | Val | Asn | Asn |
| 331 | Thr | Asn | Glu | Ile | Tyr | Glu | Met | Asn | Asn | Met | Asn | Asp | Val | Asn | Asn |
| 346 | Thr | Asn | Glu | Ile | Asn | Val | Val | Asn | Ala | Val | Asn | Glu | Val | Asn | Lys |
| 361 | Val | Asn | Asp | Ser | Asn | Asn | Ser | Asn | Asp | Ala | Asn | Glu | Gly | Asn | Asn |
| 376 | Ala | Asn | Tyr | Ser | Asn | Asp | Ser | Ser | Asn | Thr | Asn | Asn | Asn | Thr | Ser |
| 391 | Ser | Ser | Thr | Asn | Asn | Ser | Asn | Asn | Asn | Thr | Ser | Cys | Ser | Ser | Gln |
| 406 | Asn | Thr | Thr | Thr | Ser | Ser | Glu | Asn | Asn | Asp | Ser | Leu | Glu | Asn | Lys |
| 421 | Arg | Asn | Glu | Glu | Asp | Glu | Asp | Glu | Glu | Asp | Asp | Gln | Lys | Asp | Thr |
| 436 | Gln | Lys | Glu | Lys | Asn | Asn | Leu | Glu | Gln | Glu | Asp | Met | Ser | Pro | Tyr |
| 451 | Glu | Asp | Arg | Asn | Lys | Asn | Asp | Glu | Lys | Asn | Ile | Asn | Glu | Gln | Asp |
| 466 | Lys | Phe | His | Leu | Ser | Asn | Asp | Leu | Gly | Lys | Ile | Tyr | Asp | Thr | Tyr |
| 481 | Asn | Gln | Gly | Asp | Glu | Val | Val | Val | Ser | Lys | Asn | Lys | Asp | Lys | Leu |
| 496 | Glu | Lys | His | Leu | Asn | Asp | Tyr | Lys | Ser | Tyr | Tyr | Tyr | Leu | Ser | Lys |
| 511 | Ala | Thr | Leu | Met | Asp | Lys | Ile | Gly | Glu | Ser | Gln | Asn | Asn | Asn | Asn |
| 526 | Tyr | Asn | Val | Cys | Asn | Ser | Asn | Glu | Leu | Gly | Thr | Asn | Glu | Ser | Ile |
| 541 | Lys | Thr | Asn | Ser | Asp | Gln | Asn | Asp | Asn | Val | Lys | Glu | Lys | Asn | Asp |
| 556 | Ser | Asn | Ile | Phe | Met | Lys | Met | Ile | Ile | Ile | Ile | Arg | Leu | Met | Ile |
| 571 | Met | Ile | Ile | Met | Ile | Met | Ile | Ile | Ile | Trp | Tyr | Leu | Lys | Ile | Leu |
| 586 | Gln | Asp | Lys | Ile | Ile | Trp | Arg | Asn | Lys | Lys | Val | Glu | Lys | Thr | Ser |
| 601 | Asn | Ile | Leu | Asn | Asn | Phe | Asp | Asn | Asn | Gly | Asn | Asp | Asn | Asp | Asn |
| 616 | Asp | Asn | Asp | Asp | Asn | Asn | Asp | Asn | Asp | Asn | Asn | Asn | Asn | Asn | Asn |
| 631 | Met | Asn | Asn | Gln | Tyr | Asn | Tyr | Gln | Glu | Asn | Asn | Ile | Asn | Thr | Asn |
| 646 | Tyr | Asn | Ile | Leu | Tyr | Thr | Pro | Ser | Asn | Cys | Gln | Ile | Gln | Asn | Asn |
| 661 | Ser | Tyr | Met | Asn | Thr | Asn | Glu | Met | Tyr | Gln | Pro | Leu | Tyr | Asn | Thr |
| 676 | Tyr | Pro | Ser | Asn | Arg | Ile | Gln | Glu | Asn | Ser | Thr | Ile | Asn | Asn | Asn |
| 691 | Ile | Ile | Asn | Asp | Ser | Pro | Tyr | Met | Asn | Asn | Asp | Asn | Thr | Thr | Asn |
| 706 | Asn | Thr | Phe | Ile | Ser | Gly | Met | Asn | | | | | | | |

# FIG 2

```
                10         20         30         40         50         60
                 |          |          |          |          |          |
   1 GAATTCCTCG TGATCATATG AGATATAGTT TTTATGTTGA GAATATTTGT TCAAAGAAGA
  61 TGCGTCACAA GAGAAGAAAC ATAAGCGCAT TTAATAAAAT TTTATATAAG AATTTTAAAT
 121 CAATCAAAAA ACAATTTACT ATGTTTCATC GTCCAAACAT GTTAAATTTA TCTTATGATT
 181 GGGATTCATT CCAAAAAGAT ATTACGGTAT TAGTAAACCA AATATTAACC TCTTACGAAT
 241 GGCATGGACA TGGAGCTTTT GAATCATTCG TTAAGTTATA TGGAGATTTA AGAAAAGATA
 301 TTTTAAGTCC TATACATCTA CTAAAAGTGT TGTGGAAAAT TTTAGAAATA TAGAAGAAGA
 361 CTTAGCTGAT TTAGATGAAG ATTCAACAGA AAATATTAAT GAACCTAATC ATTTAGATGG
 421 TCAAAATAAT AAAAACAATA GAAAAACTAA TAATGATAAT ACATTGAAAC AAAATCATCG
 481 AAAATCTAGG GGCACATCTG TACAAGGACG CAAAAATAAA ATAAATCGGG GATCAAAAGG
 541 CAAACATAAT TCTATAAATA TTCCAAAAGA TAGAAAGACG AACATAATGT CACAAATTAA
 601 TAAATTACTA TTTAATAAAA AAGATATTAA AATAAAATGT GAAGAAAGTA GTAGTTCAAA
 661 TTATGAAGAG GGAAATAGTT CGAGTAATGA AGAAAATAAT ATATCAACTG ATAAAAATAT
 721 TTGTAATACA AATAATAAAA ATGGAGTTTC ATTATATGAT AATTCAAAGG TTTATTTGAA
 781 AGGAGATTAT AAAATTTAGC AAACAGTTTC ATAAGTACAT ACACAAGAAC CTTTTAGATA
 841 ATGTCGATAA AACAGATAGA ACAATTAATA TTATATCAAA ATTCTTCGGT GGTGTGAATA
 901 AGTCCAATAA CGTGAATAAT ATTAATAGTG TAAATAAGGA GAATAACATG AATAAGGTGA
 961 ATGCTGTACA TAAGATAAAT GCTGTGGATA AGGTAAATGC TGTGAATAAG GTAAATTCTG
1021 TCAATAAGCT AAATGTTGTG AATAAGACGA ATGTTCTGAG TAAGTTGAAT GCTGTGTATA
1081 AGGTGAATTC TGTACATAAG ATGAATGCTG TGAATAAGGT AAATGCTGTA AATAAGGTGA
1141 ATGCTGTAAA TAAGGTAAAT GTCGTGAATA AGAAGGATAT TCTGAATAAG TTGAATGCTT
1201 TGTATAAGAT GAATGCTGTG TATAAAATGA ATGCTTTGAA TAAGGTGAGT GCTGTGAATA
1261 AGGTGAGTGC TGTGAATAAG GTGAGTGCTG TGAATAAGAT GGGTGCTGTA AATAGGGTGA
1321 ATGGAGTAAA CAAGGTGAAT GAGGTGAATG AGGTGAATGA GGTGAATGAA GTGAATATGG
1381 TGAATGAAGT AAATGAGTTA AATGAGGTGA ATAATGTCAA TGCAGTGAAT GAAGTGAATA
1441 GTGTGAACGA GGTTAATGAA ATGAATGAGG TGAATAAGGT GAATGAGCTA AATGAGGTGA
1501 ATGAAGTGAA TAATGTCAAT GAGGTGAATA ATGTGAATGT GATGAATAAT GTGAATGAGA
1561 TGAATAATAT GAATGAGATG AATAATGTCA ATGTAGTGAA TGAAGTGAAT AATGTCAATG
1621 AGGTGAATAA TGTGAATGAG ATGAATAATG TGAATGAGAT GAATAATATG AATGAGATGA
1681 ATAATGTCAA TGTAGTGAAT GAAGTGAATA ATGTAAATGA GATGAATAAT ACGAATGAGC
1741 TAAATGAGGT GAATGAAGTG AATAATGTGA ATGAGGTGAA TGATGTCAAT GTAGTGAACG
1801 AAGTGAATAA TGTAAATGAG ATGAATAATA TGAATGAGCT AAATGAGGTG AATGGGGTAA
1861 ATGAAGTGAA TAATACGAAT GAGATACATG AGATGAATAA TATAAATGAG GTGAATAATA
1921 CGAATGAGGT GAATAATACG AATGAGATAT ATGAGATGAA TAATATGAAT GATGTGAATA
1981 ATACGAATGA GATAAATGTG GTGAATGCGG TTAATGAAGT GAATAAGGTG AATGATTCAA
2041 ATAATTCAAA TGATGCAAAT GAAGGAAATA ACGCAAATTA TTCAAATGAT TCAAGCAATA
2101 CAAATAATAA CACATCAAGC AGCACAAATA ACTCAAATAA TAATACATCG TGTAGTTCAC
2161 AGAACACCAC AACTAGCAGC GAAAATAATG ATTCATTAGA AAATAAAAGA AATGAAGAAG
2221 ATGAAGATGA AGAAGACGAC CAAAAAGATA CACAAAAAGA AAAAAACAAT TTAGAACAGG
2281 AAGATATGAG TCCATACGAA GATAGAAATA AAAATGATGA AAAAAATATT AATGAACAAG
2341 ATAAATTTCA TTTATCAAAT GATTTGGGAA AAATATATGA TACATATAAC CAAGGAGATG
2401 AAGTTGTTGT ATCTAAGAAT AAGGACAAAT TAGAAAAGCA TTTGAATGAT TACAAGAGTT
2461 ATTATTATTT ATCTAAAGCA ACACTCATGG ACAAAATTGG AGAATCACAA AATAATAACA
2521 ACTATAATGT ATGTAATTCA AATGAACTTG GAACTAATGA ATCCATAAAG ACAAATTCTG
2581 ATCAGAATGA TAATGTAAAA GAAAAAAATG ATTCCAACAT ATTTATGAAA ATGATAATTA
2641 TAATTCGTCT TATGATAATG ATCATAATGA TAATGATAAT AATATGGTAT TTAAAGATTC
2701 TTCAAGACAA GATAATATGG AGAAACAAAA AAGTGGAGAA AACAAGCAAT ATTTTAAACA
2761 ATTTCGATAA TAATGGTAAT GATAATGATA ATGATAATGA TGATAATAAT GATAATGATA
2821 ATAATAATAA TAATAATATG AATAATCAAT ATAATTATCA AGAAAATAAT ATTAACACAA
2881 ATTATAACAT TTTGTACACT CCTTCTAATT GCCAAATCCA AAACAATTCA TATATGAATA
2941 CAAATGAAAT GTACCAACCA TTATATAATA CATATCCTTC AAATCGTATT CAAGAAAATT
3001 CTACTATAAA TAACAACATT ATTAATGATT CACCTTACAT GAATAACGAC AACACCACTA
3061 ATAACACCTT CATTTCTGGT ATGAATTC
```

# FIG 3

| | | | | | 5 | | | | | 10 | | | | | 15 |
|---|---|---|---|---|---|---|---|---|---|---|---|---|---|---|---|
| 1 | Met | Arg | Gly | Ser | His | His | His | His | His | His | Gly | Ser | Val | Asn | Ser |
| 16 | Val | Asn | Lys | Glu | Asn | Asn | Met | Asn | Lys | Val | Asn | Ala | Val | His | Lys |
| 31 | Ile | Asn | Ala | Val | Asp | Lys | Val | Asn | Ala | Val | Asn | Lys | Val | Asn | Ser |
| 46 | Val | Asn | Lys | Leu | Asn | Val | Val | Asn | Lys | Thr | Asn | Val | Leu | Ser | Lys |
| 61 | Leu | Asn | Ala | Val | Tyr | Lys | Val | Asn | Ser | Val | His | Lys | Met | Asn | Ala |
| 76 | Val | Asn | Lys | Val | Asn | Ala | Val | Asn | Lys | Val | Asn | Ala | Val | Asn | Lys |
| 91 | Val | Asn | Val | Val | Asn | Lys | Lys | Asp | Ile | Leu | Asn | Lys | Leu | Asn | Ala |
| 106 | Leu | Tyr | Lys | Met | Asn | Ala | Val | Tyr | Lys | Met | Asn | Ala | Leu | Asn | Lys |
| 121 | Val | Ser | Ala | Val | Asn | Lys | Val | Ser | Ala | Val | Asn | Lys | Val | Ser | Ala |
| 136 | Val | Asn | Lys | Met | Gly | Ala | Val | Asn | Arg | Val | Asn | Gly | Val | Asn | Lys |
| 151 | Val | Asn | Glu | Val | Asn | Glu | Val | Asn | Glu | Val | Asn | Glu | Val | Asn | Met |
| 166 | Val | Asn | Glu | Val | Asn | Glu | Leu | Asn | Glu | Val | Asn | Asn | Val | Asn | Ala |
| 181 | Val | Asn | Glu | Val | Asn | Ser | Val | Asn | Glu | Val | Asn | Glu | Met | Asn | Glu |
| 196 | Val | Asn | Lys | Val | Asn | Glu | Leu | Asn | Glu | Val | Asn | Glu | Val | Asn | Asn |
| 211 | Val | Asn | Glu | Val | Asn | Asn | Val | Asn | Val | Met | Asn | Asn | Val | Asn | Glu |
| 226 | Met | Asn | Asn | Met | Asn | Glu | Met | Asn | Asn | Val | Asn | Val | Val | Asn | Glu |
| 241 | Val | Asn | Asn | Val | Asn | Glu | Val | Asn | Asn | Val | Asn | Glu | Met | Asn | Asn |
| 256 | Val | Asn | Glu | Met | Asn | Asn | Met | Asn | Glu | Met | Asn | Asn | Val | Asn | Val |
| 271 | Val | Asn | Glu | Val | Asn | Asn | Val | Asn | Glu | Met | Asn | Asn | Thr | Asn | Glu |
| 286 | Leu | Asn | Glu | Val | Asn | Glu | Val | Asn | Asn | Val | Asn | Glu | Val | Asn | Asp |
| 301 | Val | Asn | Val | Val | Asn | Glu | Val | Asn | Asn | Val | Asn | Glu | Met | Asn | Asn |
| 316 | Met | Asn | Glu | Leu | Asn | Glu | Val | Asn | Gly | Val | Asn | Glu | Val | Asn | Asn |
| 331 | Thr | Asn | Glu | Ile | His | Glu | Met | Asn | Asn | Ile | Asn | Glu | Val | Asn | Asn |
| 346 | Thr | Asn | Glu | Val | Asn | Asn | Thr | Asn | Glu | Ile | Tyr | Glu | Met | Asn | Asn |
| 361 | Met | Asn | Asp | Val | Asn | Asn | Thr | Asn | Glu | Ile | Asn | Val | Val | Asn | Ala |
| 376 | Val | Asn | Glu | Val | Asn | Lys | Val | Asn | Asp | Ser | Asn | Asn | Ser | Asn | Asp |
| 391 | Ala | Asn | Glu | Gly | Asn | Asn | Ala | Asn | Tyr | Ser | Asn | Asp | Ser | Ser | Asn |
| 406 | Thr | Asn | Asn | Asn | Thr | Ser | Ser | Ser | Thr | Asn | Asn | Ser | Asn | Asn | Asn |
| 421 | Thr | Ser | Cys | Ser | Ser | Gln | Asn | Thr | Thr | Thr | Ser | Ser | Glu | Asn | Asn |
| 436 | Asp | Ser | Leu | Glu | Asn | Lys | Arg | Asn | Glu | Glu | Asp | Glu | Asp | Glu | Glu |
| 451 | Asp | Asp | Gln | Lys | Asp | Thr | Gln | Lys | Glu | Lys | Asn | Asn | Leu | Glu | Gln |
| 466 | Glu | Asp | Met | Ser | Pro | Tyr | Glu | Asp | Arg | Asn | Lys | Asn | Asp | Glu | Lys |
| 481 | Asn | Ile | Asn | Gly | Ile | Arg | Arg | Pro | Ala | Ala | Lys | Leu | Asn | | |

FIG 4

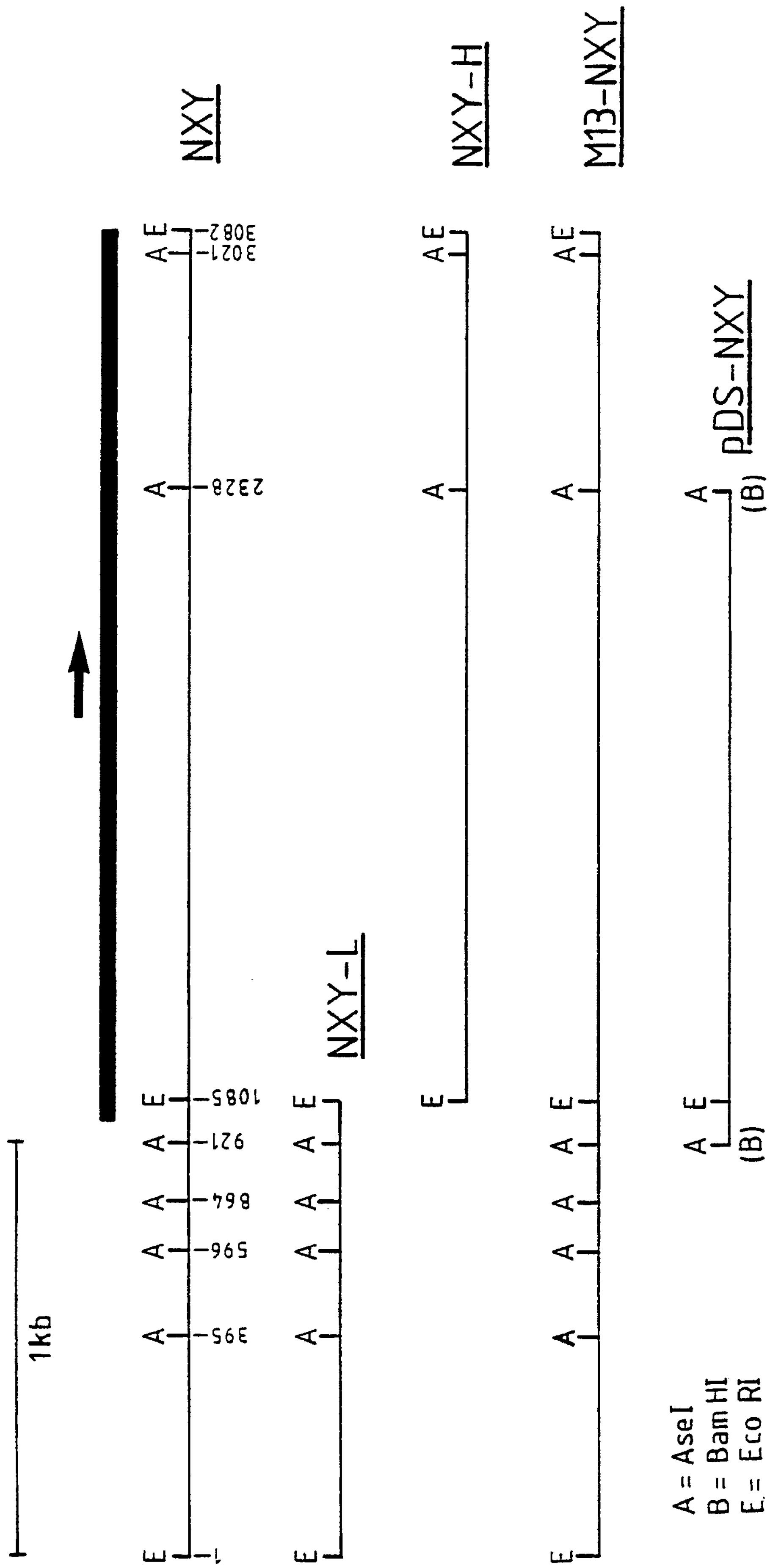

1kb
NXY
NXY-L
NXY-H
M13-NXY
pDS-NXY
1
395
596
864
921
1085
2328
3021
3082
(B)
(B)
A = AseI
B = BamHI
E = EcoRI

FIG 5

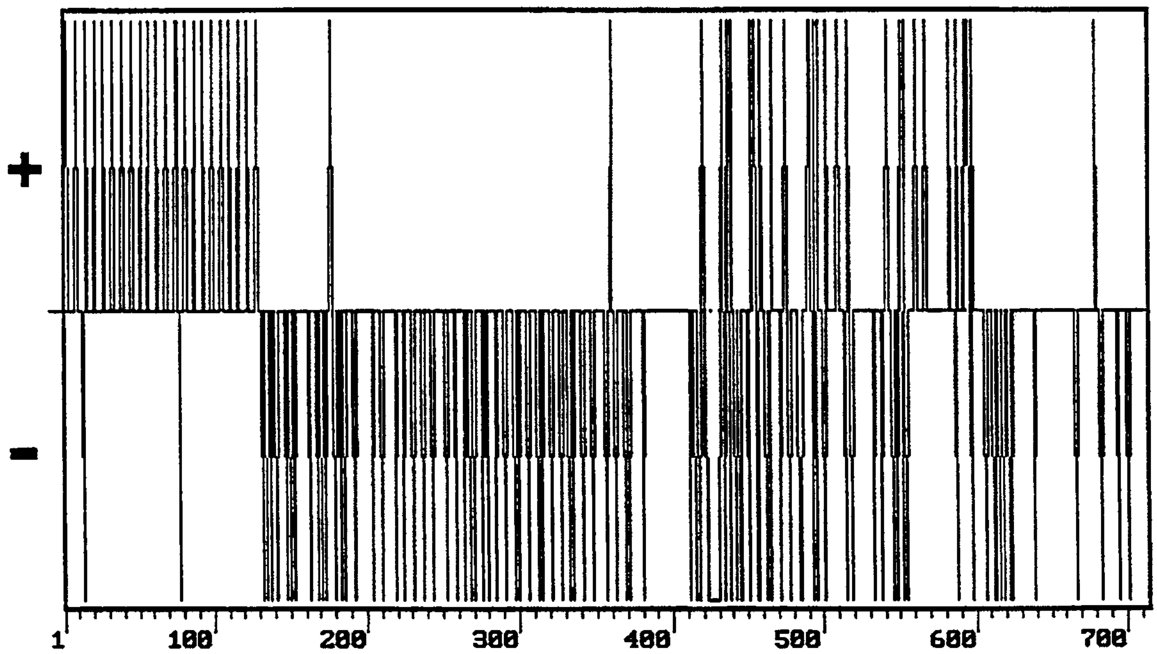

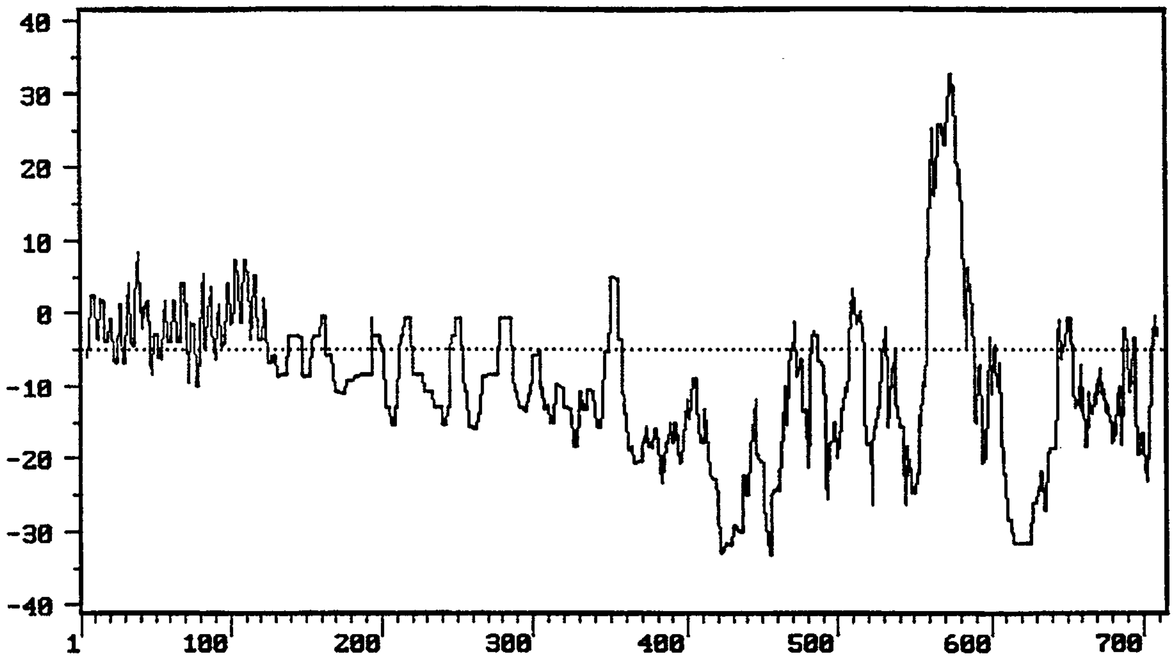
40
30
20
10
0
-10
-20
-30
-40
1
100
200
300
400
500
600
700

FIG 7

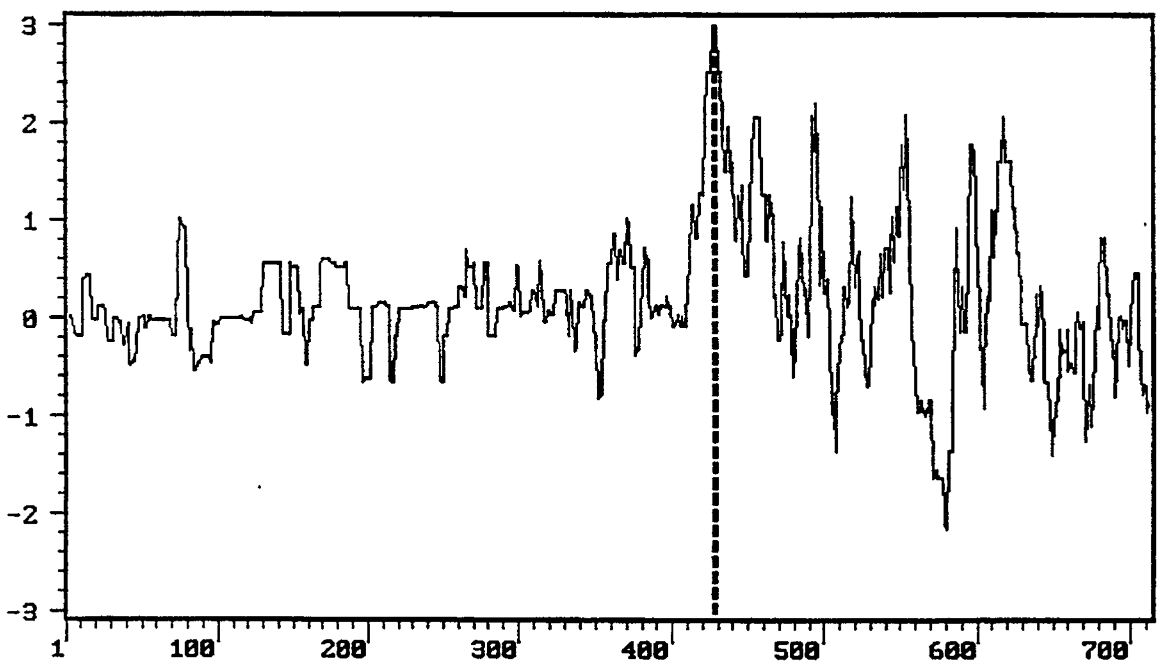

# FIG 8

A

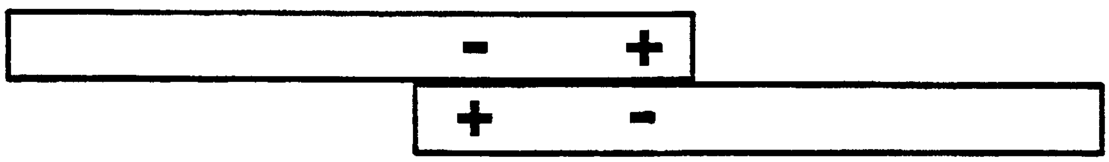


B

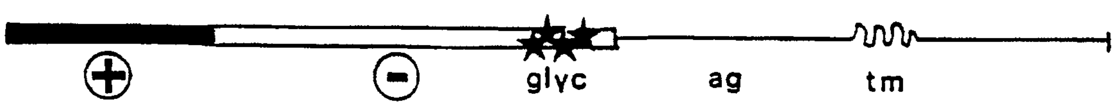

FIG 9

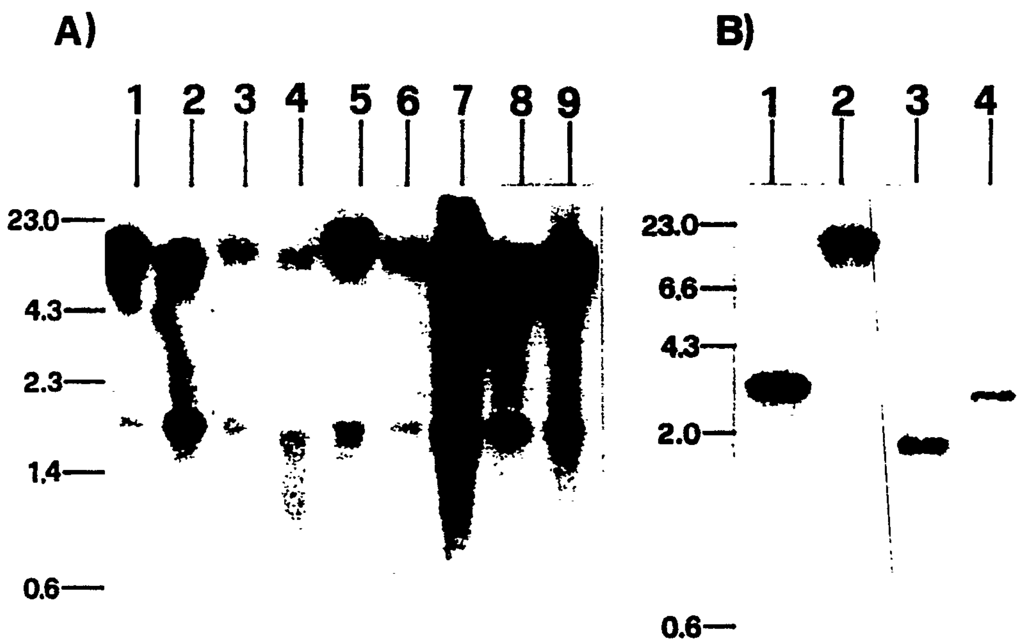

FIG. 10

```
      XhoI
   1  CTCGAGAAAT CATAAAAAAT TTATTTGCTT TGTGAGCGGA TAACAATTAT

                                                EcoRI
  51  AATAGATTCA ATTGTGAGCG GATAACAATT TCACACAGAA TTCATTAAAG

                                                     BamHI
 101  AGGAGAAATT AACTATGAGA GGATCGCATC ACCATCACCA TCACGGATCC
                     MetArg GlySerHisH isHisHisHi sHisGlySer

      SalI  PstI    HindIII
 151  GTCGACCTGC AGCCAAGCTT AATTAGCTGA GCTTGGACTC CTGTTGATAG
      ValAspLeuG lnProSerLe uIleSer

 201  ATCCAGTAAT GACCTCAGAA CTCCATCTGG ATTTGTTCAG AACGCTCGGT

 251  TGCCGCCGGG CGTTTTTTAT TGGTGAGAAT CCAAGCTAGC TTGGCGAGAT

 301  TTTCAGGAGC TAAGGAAGCT AAAATGGAGA AAAAAATCAC TGGATATACC

 351  ACCGTTGATA TATCCCAATG GCATCGTAAA GAACATTTTG AGGCATTTCA

 401  GTCAGTTGCT CAATGTACCT ATAACCAGAC CGTTCAGCTG GATATTACGG

 451  CCTTTTTAAA GACCGTAAAG AAAAATAAGC ACAAGTTTTA TCCGGCCTTT

 501  ATTCACATTC TTGCCCGCCT GATGAATGCT CATCCGGAAT TTCGTATGGC

 551  AATGAAAGAC GGTGAGCTGG TGATATGGGA TAGTGTTCAC CCTTGTTACA

 601  CCGTTTTCCA TGAGCAAACT GAAACGTTTT CATCGCTCTG GAGTGAATAC

 651  CACGACGATT TCCGGCAGTT TCTACACATA TATTCGCAAG ATGTGGCGTG

 701  TTACGGTGAA AACCTGGCCT ATTTCCCTAA AGGGTTTATT GAGAATATGT

 751  TTTTCGTCTC AGCCAATCCC TGGGTGAGTT TCACCAGTTT TGATTTAAAC

 801  GTGGCCAATA TGGACAACTT CTTCGCCCCC GTTTTCACCA TGGGCAAATA

 851  TTATACGCAA GGCGACAAGG TGCTGATGCC GCTGGCGATT CAGGTTCATC

 901  ATGCCGTCTG TGATGGCTTC CATGTCGGCA GAATGCTTAA TGAATTACAA

 951  CAGTACTGCG ATGAGTGGCA GGGCGGGGCG TAATTTTTTT AAGGCAGTTA

1001  TTGGTGCCCT TAAACGCCTG GGGTAATGAC TCTCTAGCTT GAGGCATCAA

1051  ATAAAACGAA AGGCTCAGTC GAAAGACTGG GCCTTTCGTT TTATCTGTTG
```

FIG. 10 (cont.)

XbaI

1101 TTTGTCGGTG AACGCTCTCC TGAGTAGGAC AAATCCGCCG CTCTAGAGCT

1151 GCCTCGCGCG TTTCGGTGAT GACGGTGAAA ACCTCTGACA CATGCAGCTC

1201 CCGGAGACGG TCACAGCTTG TCTGTAAGCG GATGCCGGGA GCAGACAAGC

1251 CCGTCAGGGC GCGTCAGCGG GTGTTGGCGG GTGTCGGGGC GCAGCCATGA

1301 CCCAGTCACG TAGCGATAGC GGAGTGTATA CTGGCTTAAC TATGCGGCAT

1351 CAGAGCAGAT TGTACTGAGA GTGCACCATA TGCGGTGTGA AATACCGCAC

1401 AGATGCGTAA GGAGAAAATA CCGCATCAGG CGCTCTTCCG CTTCCTCGCT

1451 CACTGACTCG CTGCGCTCGG TCTGTCGGCT GCGGCGAGCG GTATCAGCTC

1501 ACTCAAAGGC GGTAATACGG TTATCCACAG AATCAGGGGA TAACGCAGGA

1551 AAGAACATGT GAGCAAAAGG CCAGCAAAAG GCCAGGAACC GTAAAAAGGC

1601 CGCGTTGCTG GCGTTTTTCC ATAGGCTCCG CCCCCCTGAC GAGCATCACA

1651 AAAATCGACG CTCAAGTCAG AGGTGGCGAA ACCCGACAGG ACTATAAAGA

1701 TACCAGGCGT TTCCCCCTGG AAGCTCCCTC GTGCGCTCTC CTGTTCCGAC

1751 CCTGCCGCTT ACCGGATACC TGTCCGCCTT TCTCCCTTCG GGAAGCGTGG

1801 CGCTTTCTCA ATGCTCACGC TGTAGGTATC TCAGTTCGGT GTAGGTCGTT

1851 CGCTCCAAGC TGGGCTGTGT GCACGAACCC CCCGTTCAGC CCGACCGCTG

1901 CGCCTTATCC GGTAACTATC GTCTTGAGTC CAACCCGGTA AGACACGACT

1951 TATCGCCACT GGCAGCAGCC ACTGGTAACA GGATTAGCAG AGCGAGGTAT

2001 GTAGGCGGTG CTACAGAGTT CTTGAAGTGG TGGCCTAACT ACGGCTACAC

2051 TAGAAGGACA GTATTTGGTA TCTGCGCTCT GCTGAAGCCA GTTACCTTCG

2101 GAAAAAGAGT TGGTAGCTCT TGATCCGGCA AACAAACCAC CGCTGGTAGC

2151 GGTGGTTTTT TTGTTTGCAA GCAGCAGATT ACGCGCAGAA AAAAAGGATC

2201 TCAAGAAGAT CCTTTGATCT TTTCTACGGG GTCTGACGCT CAGTGGAACG

2251 AAAACTCACG TTAAGGGATT TTGGTCATGA GATTATCAAA AAGGATCTTC

FIG. 10 (cont.)

```
2301  ACCTAGATCC TTTTAAATTA AAAATGAAGT TTTAAATCAA TCTAAAGTAT
2351  ATATGAGTAA ACTTGGTCTG ACAGTTACCA ATGCTTAATC AGTGAGGCAC
2401  CTATCTCAGC GATCTGTCTA TTTCGTTCAT CCATAGCTGC CTGACTCCCC
2451  GTCGTGTAGA TAACTACGAT ACGGGAGGGC TTACCATCTG GCCCCAGTGC
2501  TGCAATGATA CCGCGAGACC CACGCTCACC GGCTCCAGAT TTATCAGCAA
2551  TAAACCAGCC AGCCGGAAGG GCCGAGCGCA GAAGTGGTCC TGCAACTTTA
2601  TCCGCCTCCA TCCAGTCTAT TAATTGTTGC CGGGAAGCTA GAGTAAGTAG
2651  TTCGCCAGTT AATAGTTTGC GCAACGTTGT TGCCATTGCT ACAGGCATCG
2701  TGGTGTCACG CTCGTCGTTT GGTATGGCTT CATTCAGCTC CGGTTCCCAA
2751  CGATCAAGGC GAGTTACATG ATCCCCCATG TTGTGCAAAA AAGCGGTTAG
2801  CTCCTTCGGT CCTCCGATCG TTGTCAGAAG TAAGTTGGCC GCAGTGTTAT
2851  CACTCATGGT TATGGCAGCA CTGCATAATT CTCTTACTGT CATGCCATCC
2901  GTAAGATGCT TTTCTGTGAC TGGTGAGTAC TCAACCAAGT CATTCTGAGA
2951  ATAGTGTATG CGGCGACCGA GTTGCTCTTG CCCGGCGTCA ATACGGGATA
3001  ATACCGCGCC ACATAGCAGA ACTTTAAAAG TGCTCATCAT TGGAAAACGT
3051  TCTTCGGGGC GAAAACTCTC AAGGATCTTA CCGCTGTTGA GATCCAGTTC
3101  GATGTAACCC ACTCGTGCAC CCAACTGATC TTCAGCATCT TTTACTTTCA
3151  CCAGCGTTTC TGGGTGAGCA AAAACAGGAA GGCAAAATGC CGCAAAAAAG
3201  GGAATAAGGG CGACACGGAA ATGTTGAATA CTCATACTCT TCCTTTTTCA
3251  ATATTATTGA AGCATTTATC AGGGTTATTG TCTCATGAGC GGATACATAT
3301  TTGAATGTAT TTAGAAAAAT AAACAAATAG GGGTTCCGCG CACATTTCCC
3351  CGAAAAGTGC CACCTGACGT CTAAGAAACC ATTATTATCA TGACATTAAC
3401  CTATAAAAAT AGGCGTATCA CGAGGCCCTT TCGTCTTCAC
```

Europäisches Patentamt

**EUROPÄISCHER TEILRECHERCHENBERICHT,**
der nach Regel 45 des Europäischen Patentübereinkommens für das weitere Verfahren als europäischer Recherchenbericht gilt

Nummer der Anmeldung
EP 91103900.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP - A2 - 0 329 257 (MICROGENESYS) * Zusammenfassung * -- | 1,15-17 | C 12 N 15/30<br>C 07 K 15/00<br>C 12 N 1/21<br>C 12 P 21/00<br>A 61 K 39/015 |
| | EP - A1 - 0 278 941 (SMITH KLINE) * Ansprüche * -- | 1,15-17 | |
| | EP - A1 - 0 250 261 (THE WELLCOME FOUNDATION) * Ansprüche * -- | 1,15-17 | |
| A | EP - A1 - 0 192 626 (SMITH KLINE BECKMAN) * Ansprüche * ---- | 1,15-17 | |

RECHERCHIERTE SACHGEBIETE (Int. Cl.5)

C 12 N
C 07 K
C 12 P
A 61 K

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-17
Unvollständig recherchierte Patentansprüche: -
Nicht recherchierte Patentansprüche: 18
Grund für die Beschränkung der Recherche: Art. 52(4)EPÜ; Verfahren zur therapeutischen Behandlung des menschlichen oder tierischen Körpers

| Recherchenort | Abschlußdatum der Recherche | Prüfer |
|---|---|---|
| WIEN | 01-07-1991 | WOLF |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1505.1 03 82